**Europäisches Patentamt**

**European Patent Office**

**Office européen des brevets**

(19)

(11) Publication number : **0 207 648 B2**

# NEW EUROPEAN PATENT SPECIFICATION

(12)

(45) Date of publication of the new patent specification : **19.05.93 Bulletin 93/20**

(51) Int. Cl.⁵ : **A61F 9/00**

(21) Application number : **86304315.4**

(22) Date of filing : **05.06.86**

(54) Apparatus for ophthalmological surgery.

(30) Priority : **06.06.85 US 742225**
**23.09.85 US 778801**

(43) Date of publication of application :
**07.01.87 Bulletin 87/02**

(45) Publication of the grant of the patent :
**16.08.90 Bulletin 90/33**

(45) Mention of the opposition decision :
**19.05.93 Bulletin 93/20**

(84) Designated Contracting States :
**BE CH DE FR GB IT LI NL SE**

(56) References cited :
**EP-A- 0 111 060**
**EP-A- 0 116 933**
**EP-A- 0 151 869**
**EP-B- 0 191 688**
**DE-A- 3 148 748**
**FR-A- 8 501 614**

(56) References cited :
**GB-A- 2 144 561**
**US-A- 3 703 176**
**US-A- 3 769 963**
**US-A- 4 461 294**
**"Excimer Laser Surgery of The Cornea",**
**S.L.Trokel et al. American Journal of Ophthal-**
**mology, Vol 96, No. 6 December 1983, pp**
**710-715**
**Brockhaus "Naturwissenschaften und**
**Technik", Wiesbaden, Band 5, SO-Z, 1983, S.**
**189**

(73) Proprietor : **VISX INCORPORATED**
**919 Kifer Road, Suite 202**
**Sunnyvale, CA 94086 (US)**

(72) Inventor : **VISX INCORPORATED**
**919 Kifer Road, Suite 202**
**Sunnyvale, CA 94086 (US)**

(74) Representative : **Milhench, Howard Leslie et al**
**R.G.C. Jenkins & Co. 26 Caxton Street**
**London SW1H 0RJ (GB)**

EP 0 207 648 B2

## Description

## FIELD OF THE INVENTION

The invention relates to that aspect of ophthalomogical surgery which is concerned with operations upon the external surface of the cornea. The apparatus of the invention has application to corneal surgery upon the eye of a living patient and also has application to donor surgery where the invention can be applied to treatment of the receptor and/or the donor tissue. The apparatus of the invention has application furthermore to corneal operations performed with the cornea in place in the eye or with the cornea removed from the eye.

Operations of the character indicated include corneal transplants and keratotomies; such operations have traditionally required skilled manipulation of a cutting instrument. But, however keen the cutting edge, the mere entry of the edge into the surface of the cornea necessarily means a wedge-like lateral pressure against body cells displaced by the entry, on both sides of the entry. Such lateral pressure is damaging to several layers of cells on both sides of the entry, to the extent impairing the ability of the wound to heal, and resulting in the formation of scar tissue.

My European patent application No 84307972 (EP-A-0151869) describes and claims a sculpture apparatus for performing ophthalmological surgery on the optically used portion of the anterior surface of the cornea, such apparatus comprising: laser means adapted for producing an output beam having a cross-section that at corneal impingement is small in relation to said optically used portion, said output beam being in the ultraviolet portion of the electromagnetic spectrum and being capable of achieving selective ablation of said anterior surface with penetration into the stroma to achieve a volumetric removal of corneal tissue by photodecomposition; laser control means, including a microprocessor, for controlling the operation of said laser means so as to effect a predetermined operative procedure, said laser control means including means for scan deflection of said beam for selectively impinging the beam throughout a predetermined area within said optically used portion, the irradiated flux density and exposure time of laser beam impingement at the cornea being such as to achieve for each scan an ablation depth which is but a fraction of the maximum depth of a required penetration; and said laser control means being adapted, at least in part by the programming of said microprocessor, to effect successive scans within said predetermined area whereby to achieve said required penetration by means of said successive scans.

EP-A-0151869 abovementioned also includes a background discussion of the effects of various available wavelengths of laser radiation in ophthalmological surgery and, in particular, surgery performed on the anterior surface of the cornea. It is explained that radiation at ultraviolet wavelengths is desirable by reason of its high photon energy. This energy is greatly effective on impact with tissue, in that molecules of tissue are decomposed on photon impact, resulting in tissue ablation by photodecomposition. Molecules at the irradiated surface are broken into smaller volatile fragments without heating the remaining substrate; the mechanism of the ablation is photochemical, i.e., the direct breaking of intra-molecular bonds. Photothermal and/or photocoagulation effects are neither characteristic nor observable in ablations at ultraviolet wavelengths, and cell damage adjacent the photodecomposed ablation is insignificant. The order of magnitude of this ablative process, in the case of radiation exposure at ultraviolet wavelengths (in the range of about 400 nm or less), is that an energy density of 1 joule/cm² incises to a depth of 1 micron (1 µ).

The response of a biological target to pulsed ultraviolet radiation emitted by a KrF excimer laser system was studied by J. Taboada et al and a corresponding report appears in "Health Physics", Vol. 40, May 1981, pp. 677 to 683 under the title "Response of the Corneal Epithelium to KrF Excimer Laser Pulses". A paper by the same author and entitled "Extreme Sensitivity in the Corneal Epithelium to Far UV ArF Excimer Laser Pulses" was presented to the Aerospace Medical Association 1981 Meeting in San Antonio, Texas, USA. Neither of these disclosures meets the terms of the claims that have been allowed in my European Patent application No. 84307972 abovementioned and neither meets the terms of the claims appended hereto, and both represent the results of scientific investigations made without reference to the development of a practical ophthalmological surgical tool. EP-A-0111060 discloses and claims an ablative photodecomposition apparatus for photo-etching a biological layer comprised of organic material by means of ultraviolet radiation focussed upon said layer, but again EP-A-0111060 contains no disclosure of a developed sculpture apparatus for performing ophthalmological surgery. Similarly, an article "Excimer Laser Surgery of the Cornea" by Stephen L. Trokel et al published in the American Journal of Ophthalmology, Vol. 96, No.6, pp. 710-715 in 1983 contains a report of experiments conducted by irradiation of enucleated cows eyes with the output of an excimer laser but contains no disclosure of a developed sculpture apparatus for performing ophthalmological surgery.

The apparatus that is specifically described in EP-A-0151869 abovementioned makes use of the technique of scanning a laser beam that is focussed to a spot on the cornea over the anterior surface of the cornea in such a controlled pattern as to sculpture the corneal surface and impart a new curvature thereto so as to achieve optical correction of an optically deficient eye. Whilst such an apparatus has been found in trials to be effective, the beam scanner and the scanner control means that are required in order to perform this technique are relatively complex and expensive. The present invention aims to obviate this problem or at least to provide an alternative form of ophthalmological sculpture apparatus.

According to one aspect of the present invention as set forth in claim 1 hereof there is provided an apparatus for performing opthalmological surgery by selective ablation of the anterior surface of the cornea with penetration into the stroma to achieve a volumetric removal of corneal tissue, said apparatus comprising laser means for producing an output beam preferably in the ultraviolet portion of the electromagnetic spectrum and of an intensity which is limited per unit time to ablate but a fraction of a predetermined maximum ablation depth into the stroma, means for directing said laser output beam onto the eye which is to be treated, and control means for determining the irradiated flux density and exposure time of laserbeam impingement at the cornea whereby to effect a controlled sculpturing action upon the cornea such as to alter the optical properties thereof, and said apparatus further comprising adjustment means including a zoom lens with a zoom drive provided in the laser beam path for adjustably determining the size and/or shape of the laser beam area at impingement upon the cornea, and said control means being operative upon said adjustment means for adjusting the laser beam area at impingement upon the cornea in a controlled manner in the course of a surgical treatment so that sculpturing action results from a controlled variation in the area of the cornea that is irradiated and a corresponding controlled variation in the cumulative ablative flux delivered to different areas of the cornea.

According to further aspects of the present invention as set forth in claims 6 and 15 hereof, instead of the abovementioned zoom lens and zoom drive there are respectively provided variable area masking means and actuating means for varying the area of the opening of the masking means, and reflector means and actuating means for varying the reflector area of said reflector means.

Described hereinafter are exemplary embodiments of the present invention wherein the position of an eye is effectively fixed with respect to a laser providing an output beam of ultraviolet radiation at an energy level capable of achieving controlled ablative photodecomposition of the cornea, namely, of the epithelium, Bowman's membrane, and stroma levels of the cornea. Irradiated flux density and exposure time are so controlled as to achieve desired depth of the ablation. As distinguished from the scanning procedure described in EP-A-0151869 aforementioned, a sculpturing action results from controlled change of projected laser-spot size, in the course of a given treatment, wherein spot size ranges from a maximum which covers the entire area to be treated, down to a predetermined minimum tolerable size. In one embodiment, a zoom lens in the optical path of projection is the means of changing spot size, and in other embodiments an indexible mask or mirror is employed; in all cases, the weighted allocation of time as function of spot size is such as to achieve a desired ultimate optically corrected cornea, from prior ascertainment of an optically deficient corneal curvature. Spot-size control is not only disclosed hereinafter for effecting spherical-curvature correction, but also for cylindrical correction in reduction of astigmatism; still further use is described in connection with a cornealtransplant procedure.

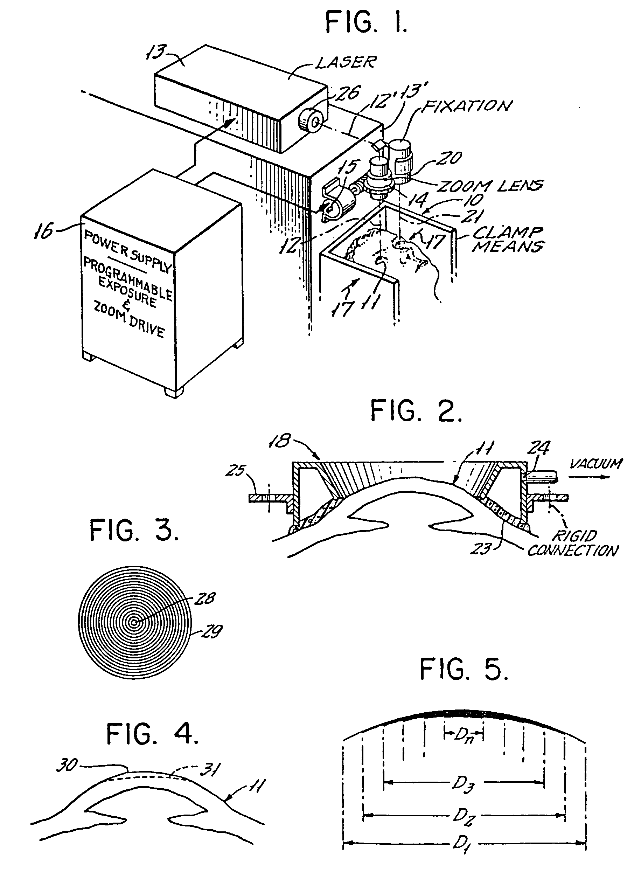

The abovementioned article by Stephen L. Trokel et al, which as has been said is merely a report of experiments conducted by irradiation, of enucleated cows eyes with the output of an excimer laser and contains no disclosure of a developed ophthalmological surgical apparatus, does contain a disclosure of the use of masks of various designs to restrict the irradiating laser energy to narrowly defined areas but contains absolutely no disclosure or suggestion that the cornea can be sculpted in an opthalmological surgical operation by exposure of the cornea to irradiation with a varying cross-section laser beam obtained by use of a series of differently sized masks. The Trokel et al article discloses "masks with a row of four slots .... and one drilled with a series of seven holes" and, as can be seen from Figs. 1 and 5 of the article the only described use made of these masks was to expose the eye to laser radiation passing through all of the apertures in the masks at the same time and for the same exposure time. The final discussion in the Trokel et al article is in vague and general terms and whilst there is mention on page 715 of application of the laser light "in a circular mask with a graded intensity from center to edge, excising more tissue either centrally or peripherally depending on the distribution of light", this does not correspond to the use in accordance with the teachings of the present invention of a series of different masks in a controlled manner, nor in Applicants expert opinion does it render such teaching obvious.

US-A-3769963, US-A-3703176 and EP-A-0116933 have been called to Applicants attention during prosecution of the present application. US-A-3769963 discloses an instrument for performing laser micro-surgery and diagnostic transillumination of living human tissue and, whilst there is disclosure of the use of laser radiation in the ultraviolet range, there is no disclosure or suggestion in US-A-3769963 of a developed ophthalmological corneal sculpture apparatus according to the teachings of the present invention. US-A-3703176 discloses a laser coagulator which incorporates a zoom lens for enabling the spot size of the laser beam to be set as desired; there is no disclosure or suggestion in US-A-3703176 of con- trollably adjusting the laser beam cross-section during an ophthalmological surgical operation so as to effect sculpture of the cornea. EP-A-0116933 discloses a laser beam machining apparatus belonging to a field of technology entirely different to that of the present invention and, whilst there is disclosure in EP-A-0116933 of the use of reflectors for setting the size and shape of a laser beam, there is no suggestion of varying the beam cross-section during a machining operation. None of these prior art references, whether considered alone or in combination, is considered relevant to the present invention.

Spot-size variation as a technique for ophthalmological surgery is disclosed in EP-A-0191688 which was not published until after the priority date of the present application, though it does itself claim an earlier priority date. The disclosure of EP-A-0191688 has much in common with the disclosure of the present application but there is no disclosure in EP-A-0191688 of the features recited in the appended claims, and in particular there is no disclosure of the use of a zoom lens, a variable area mask having a variable area opening, or a reflector having a variable reflector area as recited in claims 1, 6 and 15 respectively.

In addition to the apparatuses abovementioned, the present invention as claimed in claim 25 hereof also extends to a method excluding the application of this method on the living human and/or animal body, of corneal sculpture by selective ablation of the corneal surface with penetration into the stroma so as to achieve a volumetric removal of corneal tissue, said method comprising: utilizing laser means for producing an output beam preferably in the ultraviolet portion of the electromagnetic spectrum and of an intensity which is limited per unit time to ablate but a fraction of a predetermined maximum ablation depth into the stroma; directing said laser output beam through a zoom lens provided in the beam path onto the cornea which is to be sculpted; and controlling the irradiated flux density and exposure time of laser-beam impingement at the cornea whereby to effect a controlled sculpting action upon the cornea such as to alter the optical properties thereof, such control being effected by adjusting the zoom lens so as to adjust the size of the laser beam area at impingement upon the cornea so that sculpturing action results from a controlled variation in the area of the cornea that is irradiated and a corresponding controlled variation in the cumulative ablative flux delivered to different areas of the cornea.

The invention extends furthermore to a method substantially as above-defined but wherein, instead of using a zoom lens as claimed in claim 25, selective use is made of beam masking means as claimed in claim 26 or of beam reflection means as claimed in claim 27 to define a plurality of given different sizes and/or shapes of laser beam area at corneal impingement.

The abovementioned and other features of the present invention are as set forth with particularity in the appended claims and will become clearly understood from consideration of the following description, given with reference to the accompanying drawings, of exemplary embodiments of the invention.

In the drawings:

Fig. 1 is a schematic diagram in perspective, to show the general arrangement of operative components of a first embodiment of the invention;

Fig. 2 is a simplified view in longitudinal section, showing an eye-retaining fixture used with the apparatus of Fig. 1;

Figs. 3, 4 and 5 are simplified diagrams to illustrate the nature of ablative corneal sculpture, performed with apparatus as in Fig. 1, for the case of correcting a myopia condition;

Fig. 6 is a simplified diagram schematically showing operative components of another embodiment of the invention;

Fig. 7 is a plan view of an indexible mask usable in the embodiment of Fig. 6;

Fig. 8 is a diagram similar to Fig. 6, to show a modification;

Fig. 9 is a fragmentary plan view of an indexible mask usable in the modification of Fig. 8;

Figs. 10 and 11 are simplified diagrams to illustrate use of the invention, for the case of correcting a hyperopia condition;

Figs. 12, 13 and 14 are simplified diagrams to illustrate use of the invention to achieve a Fresnel-type optically corrective contour at the anterior surface of the cornea;

Figs. 15 and 16 respectively illustrate components and features of an embodiment of the invention to achieve correction of an astigmatic eye;

Figs. 17 and 18 are simplified diagrams to illustrate use of the invention in connection with a corneal-transplant operation;

Figs. 19 and 20 are simplified diagrams to illustrate two different alternatives for the embodiment of Figs. 15 and 16;

Figs. 21 to 26 correspond to Figs. 6, 7, 8, 9, 11 and 14, respectively, in illustration of a further aspect of the invention; and

Figs. 27 and 28 are graphical diagrams to illustrate a principle of reflector design.

In Fig. 1, clamp means 10 is shown for fixed retention of the head of a patient (reclined, face up) such that the eye 11 to be operated upon is fixedly aligned with a downwardly folded portion 12 of the central axis 12' of beam output from a stationary laser device 13, supported by a table or other base 13'. The optical system of laser-beam projection to eye 11 includes zoom-lens means 14 having a reversible motor drive 15, whereby laser-spot size at eye 11 can be caused to vary from a predetermined minimum, to a maximum of 3 or 3.5-mm radius, corresponding to the corneal frontal area to be subjected to laser action. A cabinet 16 is shown by legend to include a power supply for the laser, and cabinet 16 is also shown (by legend) to include programmable microprocessor means for controlling exposure and beam (spot) size on axis 12, as will later become more clear.

Clamp means 10 preferably includes means, symbolized at 17, to stabilize the patient's head via opposed engagements at the region of his temples, and an eye-retaining fixture (18, in Fig. 2) peripherally engages eye 11 at the corneal-scleral area. Also preferably, an optical-fixation device 20 is adjustably fixed, as to the table or base 13'. Illustratively, device 20 includes a sighting reticle and lens, whereby the eye 11' not being operated upon can view the reticle as if at infinity; the sighting alignment 21 for device 20 is parallel to the axis 12, and it will be understood that adjustable means (not shown) may provide an adjustable offset, as needed for accommodation of the patient's inter-pupilary distance and to adapt to the particular mounted offset of device 20 from axis 12. For an operation on the other eye 11', the eye 11 will be available for similar fixation, in conjunction with another fixation device (not shown) and associated adjustably offsetting means; alternatively, the fixation device 30 may be adjustably mounted at correct offset on the opposite side of scanner 14. For purposes of operating on eye 11', clamp means 10 will have been indexed laterally with respect to laser 13 to the extent aligning axis 12 with the eye (11') then to be operated upon, thereby positioning eye 11 for use of the fixation device.

The eye-retaining fixture 18 of Fig. 2 is seen to comprise a hollow annulus, having a convergent axial-end wall 23 of air-permeable material contoured to engage and retain the eye via a scleral-corneal region. A side-port connection 24 to a vacuum pump enables retention of eye engagement to wall 23, and outward lug or flange means 25 enables rigid aligned and spaced connection of fixture 18 to laser 13 and its scanner 14 via means suggested by legend in Fig. 2, such means being omitted from Fig. 1 for reasons of more simplified showing.

The laser selected for use at 13 preferably emits in the ultraviolet, namely, at wavelengths of less than substantially 400 nanometers. Such emissions for gas lasers are characteristically at 351-nm for xenon-fluoride lasers, 337-nm for nitrogen lasers, 308-nm for xenon-chloride lasers, 248-nm for krypton-fluoride lasers, 193-nm for argon fluoride lasers, and 157-nm for fluorine lasers; and within this range, frequency-doubling techniques applied to other lasers, including crystal lasers, provide further alternative sources.

One of the existing commercial excimer-laser products of Lambda Physik GmbH, Gottingen, Germany, for example their Model EMG 103 operating with argon-fluoride, is satisfactory for use as laser 13; for this product, maximum energy per pulse is 200 milli-joules, with a pulse-repetition rate of 200 per second, $3 \times 10^5$ shots (pulses) being available from a single charge of the involved gas, before reducing to 50 percent of specified power at this repetition rate, it being noted that full rated power is not necessarily required in use of the present invention. Pulse width is about 15 nanoseconds, and typical beam dimensions are rectangular; as shown, however, the opening in a mask 26 reduces the laser beam to a circular section, and it will be understood that the optical elements of lens 14 are of quartz, calcium fluoride, magnesium fluoride, or otherwise as suitable for laser-beam accommodation.

Fig. 3 is an attempt to depict the action of laser output as modified by the setting of zoom lens 14, it having already been indicated that, through the action of lens 14, spot size at eye 11 can be caused to vary from a minimum diameter at 28 to a maximum diameter at 29. The diagram shows a plurality of intermediate circular spot sizes, but it will be understood that since the zoom adjustment of lens 14 is continuously variable, there is no need to presuppose discrete circular spots of different diameter, except for the fact that in the course of a continuous variation in zoom adjustment the intermittent delivery of laser pulses will mean that each pulse is projected at a slightly different spot size.

Figs. 4 and 5 are illustrative of use of the invention in an optically corrective ablation of the anterior surface 30 of eye 11, wherein a myopia problem is to be solved, meaning that the curvature of surface 30 is of too-short radius to establish focus at the retina, for the case of distant objects. On the other hand, the dashed line 31 represents the ultimate curvature to which the anterior surface of the cornea should be modified to achieve a diopter reducing corrective effect. To achieve the curve 31, the minimum desired photodecomposition is at the outer boundary 29, and the maximum is at the center. This is achievable by programming the microprocessor to progressively change the projected spot size (through driven adjustment of lens 14) in the course of a predetermined succession of laser pulses. The result is the same whether spot size is caused to expand from minimum (28) to maximum (29) or to reduce from maximum (29) to minimum (28). Of course, for each laser pulse or "shot", ablative penetration into the cornea will be a function of delivered energy density, and therefore the number of pulses needed to achieve a given increment of ablative penetration will be greater, the larger the diameter of the projected spot.

Fig. 5 is a very much simplified diagram to represent the progressive ablative effect of a succession of laser-spot projections at successively reducing diameters $D_1$, $D_2$, $D_3$ ... $D_n$. The least resulting energy density is at the largest diameter $D_1$, which can be assumed to have made the least penetration, although such penetration will have been uniform over the entire spot area for diameter $D_1$. An incrementally greater energy density results at the next step $D_2$ of diameter reduction, in which event penetration has become cummulative with that of the first shot, over the area common to both shots. The cummulative penetration effect continues for shots of successively reduced diameter, so that a new, larger-radius curvature emerges from a pattern of stepped reduction in projected spot size. However, for a sufficiently great number of laser pulses (and hence, potentially discrete steps), individual steps cease to appear discrete, and a sufficiently smooth new spherical anterior surface characterizes the cornea. This is particularly so after a post-operative period of about two days, by which time a thin epithelial layer will have spread into smooth and protective coverage of the newly characterized surface.

The foregoing discussion in connection with Figs. 1 to 5 presupposes a pulsed laser, exemplified by an excimer laser. But other lasers are known to emit at presently suitably energy levels and at ultraviolet wavelengths of present utility, and these other lasers will emit continuously for periods of controlled duration. For example, an organic-dye laser utilizing the proper organic dye can be made to produce laser emission in the region of 380-nm when pumped by ultraviolet laser sources such as a continuous-wave frequency-quadrupled neodymium-YAG laser operating at 266-nm; in this case, the organic-dye laser emission at 380-nm can be frequency-doubled by a proper nonlinear crystal such as a potassium-deuterium-phosphate (KDP) crystal or a potassium-titanium-phosphate (KTP) crystal to an emission wavelength at 190-nm. The showing of Figs. 1 to 5 will thus be understood to illustrate the further case wherein ultraviolet laser radiation on axis 12 is of continuous-wave nature, for a treatment duration predetermined by programming at 16, and wherein the programming at 16 further continuously drives the zoom-lens 14 to provide that time-variation of projected spot size as has been predetermined to achieve a myopia-correcting change in curvature, from curve 30 to curve 31, in the course of the treatment duration. And this result is achieved whether spot size (at the eye 11) is caused to expand continuously from minimum (28) to maximum (29) or to reduce continuously from maximum (29) to minimum (28).

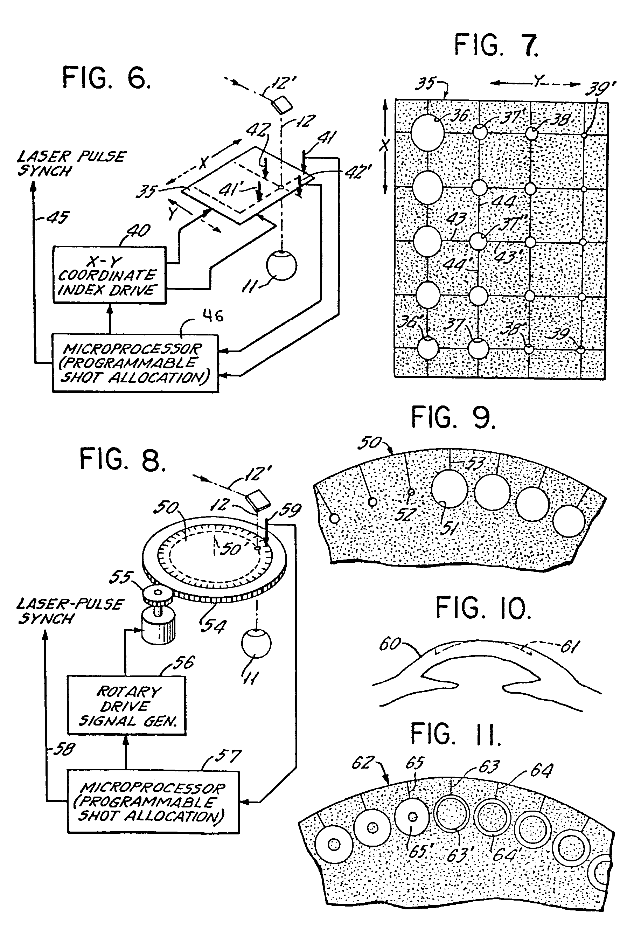

In the embodiment of Figs. 6 and 7, a masking technique is employed, in place of the zoom-lens technique of Fig. 1, to achieve a similar myopia-correcting curvature change in the anterior surface of the cornea. Such masking could proceed continuously with a suitably programmed variable iris diaphragm in place of lens 14, but in the form shown, a single precision masking plate 35 is employed. The masking plate 35 is rectangular and is mounted (by means not shown) for indexed unit displacement in each or both of two orthogonal axes X-Y. For each of the grid-like layouts of mask openings provided on plate 35, the size of the involved circular opening incrementally changes. Thus, for a first row of mask openings beginning and ending with openings 36 and 36', respectively, the openings are of progressively reducing diameter; in the next-adjacent row, beginning and ending with openings 37 and 37', respectively, the openings continue with progressively reducing diameter; in the third row, the progression continues to reduce from opening 38 to opening 38', and the final row reduces still further from 39 to the smallest opening 39'. An X-Y coordinate index drive 40 will be understood to provide correct X and/or Y successive displacements of masking plate 35 under control of microprocessor means 46 having programmable means for allocating numbers of excimer-laser "shots" (or, in the case of a CW laser, for allocating variously controlled pulse duration) at particular succeeding mask-opening sizes, whereby to effect a given desired ablative "sculpture" which will predictably and correctively change optical performance of the eye (11). As shown, optical-transducer elements in pairs 41-41' and 42-42' straddle each mask opening as it is indexed into the laser-projection axis 12; these transducer elements sense registry with grid lines, such as x-positioning grid lines 43-43' on opposite sides of a given mask opening 37" (Fig. 7) and orthogonally related y-positioning grid lines 44-44' on opposite sides of the same mask opening 37", whereby such registry may be certified to the microprocessor 46, for interlock purposes to, achieve correct mask-opening positioning on axis 12 before firing the next laser pulse, the latter being symbolized by a synchronizing connection 45.

In the arrangement of Figs. 8 and 9 myopia-correcting sculpture relies on indexed shifting from one to another of successive different-area mask openings, via incremental angular indexing displacement of a masking disc 50 (about an indexing axis 50'); disc 50 has a peripherally distributed succession of mask openings, ranging from the largest opening 51 to the smallest opening 52. A radial mark, as at 53 for opening 51, identifies the angle at which the given opening is correctly indexed into position on the laser-projection axis 12. Disc 50 is shown mounted to an annular ring 54 which will be understood to be counterbored for central and keyed location of disc 50, and ring 54 is edge-driven by suitable means 55 under control of a rotary-drive signal generator 56. Again, a programmable micro-processor 57 is responsible for controlling the rotary-index drive 55-56 for predetermined allocation of laser pulses to given mask openings, to achieve the desired cornea-profile correction, with laser-pulse synchronization via lines 58, as an optical transducer 59 tracks registry with the particular radial-marker line for each given mask-opening area.

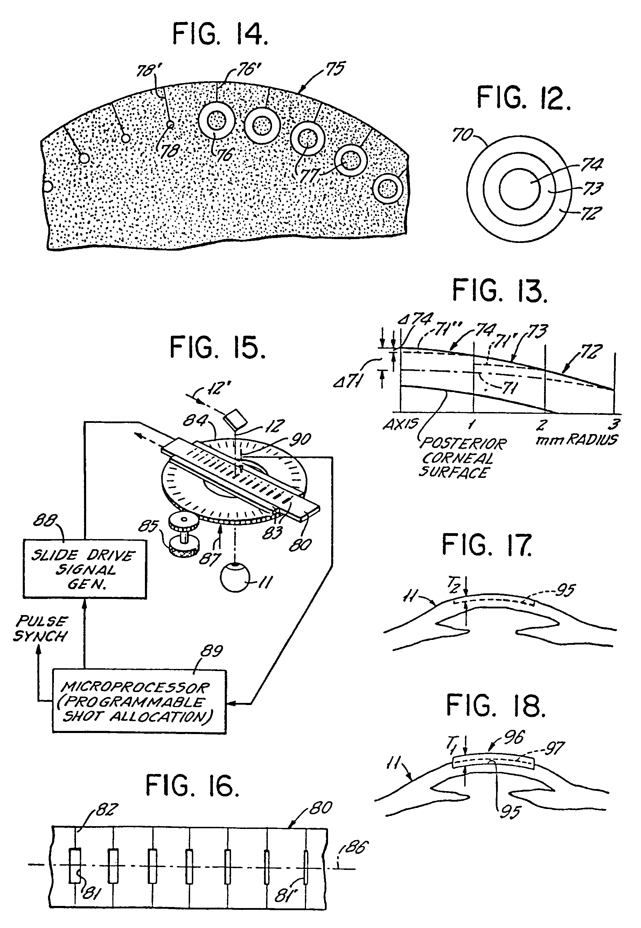

Figs. 10 and 11 illustrate that the device of Fig. 8 is equally adaptable to making corrective sculpture of the cornea 60 of a far-sighted eye, meaning that the anterior curvature is to be increased, as to achieve a new profile 61 (Fig. 10). This is illustratively done by substituting a different masking disc 62 for the disc 50 of Fig. 8. In the disc 62, for each of the angular mark locations (as at 63), a basic opening limit, eg, of 3.5-mm radius, is the outer edge of each of an angularly distributed succession of annulus openings, produced by a central opaque masking spot of progressively changing diameter. Thus, for the smallest annular mask area 63' (which applies at radial mark 63), the central opaque spot is a circle of nearly the diameter of the basic limiting opening, to produce a first, or thinnest annulus 63'. At the next mark 64, the outer diameter of a slightly thicker annulus 64' is determined by a central opaque spot of slightly lesser area. The progression continues, at increments of equal angle (about the index axis of disc 62), until reaching the largest annular opening 65' at angular location 65, where the central opaque masking circle is of least diameter. In use of the mask 62 in conjunction with the positioning and control apparatus of Fig. 8, the microprocessor 57 will be understood to so allocate laser pulses to particular sizes of annular mask openings that greatest cummulative ablative penetration of the cornea is at larger radii, while least penetration is at smaller radii, resulting in the corrected ultimate profile 61 of decreased radius.

The arrangement of Figs. 12, 13 and 14 illustrates that above-discussed principles of the invention are further applicable to corrective sculpture of the cornea to achieve a Fresnel-type distribution of the desired ultimate curvature, which can be either hyperopia-correcting or, as shown, myopia-correcting. Such an operation (i.e., Fresnel-type) would be used when, in the surgeon's considered judgment, a single smoothly developed corrected curvature would entail excessive removal of tissue at the involved region of necessarily deepest cut. To avoid too deep a cut, Figs. 12 and 13 illustrate that an ultimately reduced-curvature surface, as at 31 in Fig. 4 (dashed line 71 in Fig. 13), is achieved in annular increments within the field bounded at 70. In the outer one of these annuli (72), the curvature and depth of cut are precisely as would have applied to generate the continuous curve 71 (i.e., without Fresnel steps). But the intermediate annular area 73 effectively achieves a continuation of curve 71 with much less volume of corneal excision. Finally, the inner circular area 74 effectively completes curve 71, with minimal removal of corneal tissue.

The removal of tissue at the center is denoted Δ74 for the Fresnel cut 74 of Figs. 12 and 13 and, comparatively, is but a small fraction of the maximum removal depth Δ71 which would have been needed to achieve the same optical correction with the smoothly developed corrected single-curvature surface 71. Fig. 14 illustrates an indexible rotary masking disc 75 of a type compatible with the system of Fig. 8, in substitution for the disc 50 of Fig. 8, to achieve Fresnel-type cuts of the nature described for different annuli 72, 73, 74. Beginning with the largest area of mask annulus 76 (at location 76') and proceeding for a first 120° sector of disc 75, the succession of annular mask openings will be understood to progress with decreasing radius, by reason of a constant-area central mask spot, in the context of a progressively shrinking outer-circle diameter. The programmable means 57 (of Fig. 8) will be understood to function as a control for allocation of laser-pulse shots, using a programmed distribution of the annular mask openings of this first sector, for achievement of the curvature 71 within outer annulus 72. A similar succession of annular mask openings will be understood to be similarly accessible via a second sector (not shown) of mask disc 75, in establishing the curvature 71' within the intermediate annulus 73. And finally, the curvature 71" is established within the inner circular area 74 by programmed projection of laser shots on axis 12, through an indexibly available succession of progressively shrinking circular openings, beginning with a mask-opening diameter of largest (circle-74) area, and reducing throughout the third sector to the smallest opening 78 at location 78', adjacent the location 76' (of the first sector).

The diagrams of Figs. 15 and 16 are illustrative of the variable aperture or indexible-mask technique of the invention in the development of corrections for astigmatism, by ablative laser pulsing with a rectangular beam section wherein the width of the section is changed to create a cylindrical profile of cummulative ablative penetration. This can be done by masking the laser beam with a slit or diaphragm of variable width, and with the ability to selectively rotate the orientation at which the major dimension of the slit is positioned (i.e., based on prior measurement of the angle and of the cylindrical diopter strength of the particular eye's astigmatism; however, in the form shown in Fig. 15, the mask is an elongate strip 80 having a succession of rectangular slit openings of progressively different width. In the fragmentary showing of Fig. 16, these openings proceed from a largest area opening 81 to a smallest area opening 81', and the central axis of symmetry of each of these openings is identified with a mark, as at 82 for opening 81; preferably, all such marks are at equal spacing.

Strip 80 is a slide guided by means 83 forming part of a rotatable mask-supporting disc or ring 84; and guide means 83 locates the longitudinal axis 86 of slot symmetry on a diameter of ring 84. Manually operable means 85 has edge-drive coupling to ring 84 to enable selective angular orientation of strip 80 (about the laser-projection axis 12), as by observation via a fixed indicator mark 87 against azimuth edge markings on ring 84. A bidirectional slide-drive signal generator 88 is under control of a microprocessor 89 to coordinate slide (80) positioning with laser-pulse control, suitably synchronized by optical-transducer (90) tracking of the mark (82) applicable to the particular indexed mask opening, whereby each mask opening can be assuredly on the axis 12 of laser-beam projection.

In use of the invention for laser surgery upon an eye having need for both astigmatic and spherical correction, it is preferred that the astigmatic correction, described in connection with Figs. 15 and 16, be the first of two procedures. This is considered advantageous because astigmatic errors are generally not as severe as spherical errors, so that fewer diopters of cylindrical curvature ablation will be involved than for the subsequent spherical-correction procedure. Furthermore, to have eliminated or substantially eliminated the astigmatism in a first procedure is to have constituted the anterior surface of the cornea to an essentially spherical surface, which (be it myopic or hyperopic in nature) is more assuredly correctively sculpted to the desired profile (also spherical) for emmetropia vision, particularly where, as in the case of this invention, all ablative-laser shots (whatever the currently operative mask opening) are effectively centered on the optical axis of the involved eye.

Figs. 17 and 18 and the following description will serve to illustrate the application of the teachings of the invention to corneal transplant procedures. In Fig. 17, the cornea of an eye 11 is shown as having been subjected to a succession of laser pulses which have been masked to the same area, of diameter D, e.g., 7-mm; the succession of pulsed laser shots will in such case be seen to produce a curved base or recessed-floor curvature 95 for reception and location of an implanted corneal transplant. Alternatively, in Fig. 17, the cornea of eye 11 may have been subjected to steady (CW) laser exposure of such intensity as to ablate (a) via the same mask on constant diameter D and (b) at a rate of ablative penetration for which a given duration (exposure time) of laser-beam projection will achieve the desired depth of penetration.

Having thus prepared the patient's eye for reception of the corneal insert to be implanted at and within the recess 95, a donated eye is then reversibly held to a fixture as described at 18 in Fig. 2 for preparation of the donor corneal insert. By "reversible" it is meant that, depending upon the manner of mounting flange 25, either the epithelium or the endothelium of the donated eye may be mounted for upward exposure to the laser beam 12, it being understood that for the latter situation with the donated eye, iris and other regions not needed for corneal-scleral mounting and for corneal operation will have been initially removed. A preferred procedure is first to so expose to laser action the concave inner side of the donated cornea;

such action is to an extent (achieved by timed CW exposure, or by multiple pulsed-laser shots, of a full circular field exceeding the diameter of recess 95) sufficient to remove tissue at least to a uniform depth within the donated stroma, whereupon the mounting of fixture 18 (and its partially machined corneal workpiece) is reversed, to expose to laser action the convex outer side of the donated cornea. Laser action on the outer side consists of two steps: first, timed CW exposure multiple laser pulses of the full circular field (exceeding the diameter of recess 95) thereby excising at least the epithelium and to a depth which preferably achieves a transplant thickness $T_1$ exceeding the depth $T_2$ of recess 95; second a scanner (not shown, but of the type disclosed in my European patent application, EP-A2-0151869 is operated in a line-cutting mode wherein successive laser pulses sequentially advance along the circumference of a circle designed for precise acceptance in the circular recess 95, until full severance of the circular cut-out, which then becomes the prepared transplant. Upon implanting, donated stroma is placed in full endothelium-free contact with the patient's prepared stroma, and the implant may be sutured. Later, upon removal of sutures, the outer surface of the eye 11 and its transplant 96 will have the appearance shown in Fig. 18, wherein the transplant projects beyond adjacent areas of the patient's cornea, and this projecting surface of the transplant may be reduced by above-described laser sculpting to a finish contour 97 of preferably flush marginal conformance with non-sculptured adjacent tissue of the patient's eye. It will be further understood that, subject to the surgeon's decision, such a finishing cut may be to a curvature which does or does not effect a predetermined change in optical performance of the eye.

It will be seen that the described methods and apparatus achieve all stated objects and provide readily controlled procedure for correcting eye abnormalities attributable to cornea curvature. The ablative penetration of laser beam action may be kept to a relatively harmless fraction of the thickness of the cornea, and whatever the depth of invasion, a natural body process provides protective epithelium coverage of the sculpted region, within a few days after an operation. The programmable coordination of laser-beam size and shape (circular, annular, or rectangular). In conjunction with numbers of pulses at given sizes and shapes will produce predictable and controlled changes in curvature, whereby cylindrical errors and/or spherical errors may be eliminated or substantially reduced, to the enhance comfort and convenience of the patient.

While the invention has been described in detail for various illustrative embodiments and modes, it will be understood that modifications may be made without departing from the scope of the invention. For example, what has been described above as manual means 85 to preset the angle at which astigmatic correction is to be achieved, may in fact be an automatically driven setting of the astigmatic-correction angle, wherein the angle-input data for making the automatic drive is produced by a diagnostic system or method as described in my European patent application no. 86304097.8 filed 29 May, 1986 and published as EP-A-0247260.

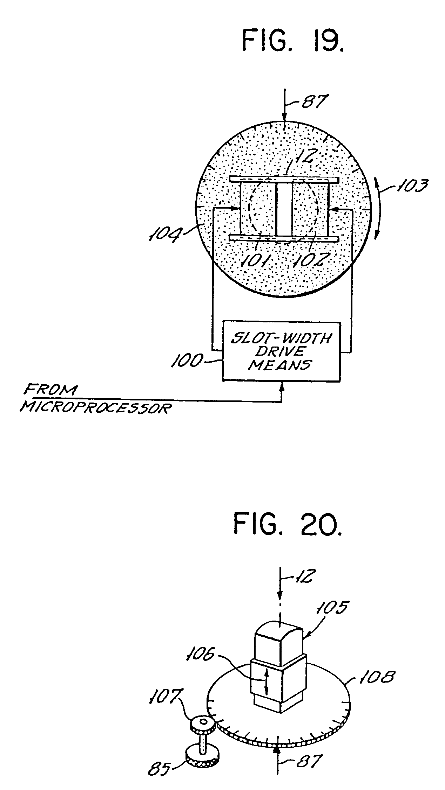

Also, by way of example, achievement of cylindrical sculpting in reduction of astigmatism does not necessarily require the indexible-slot technique of Figs. 15 and 16. As a first alternative (Fig. 19), the variation in slot width may be achieved electro-mechanically, via microprocessor control of means 100 to differentially drive opposite side plates 101-102 of a variable-width opening which is always centered on the axis of the projected laser beam 12, plates 101-102 being slidably mounted to an annular base 104 which is adjustable in rotation to the angle for which astigmatism is to be reduced (as suggested by a double arrow 103). As a second alternative (Fig. 20), a cylindrical-lens zoom system 105 is motor-driven by microprocessor output (as suggested by double arrow 106) to establish a shaping of the projected laser beam 12 to a line of variable width, and said line is settable to the angle for which astigmatism is to be reduced, as by edge-drive means 107 to the rim 108 of an annular mount for zoom system 105.

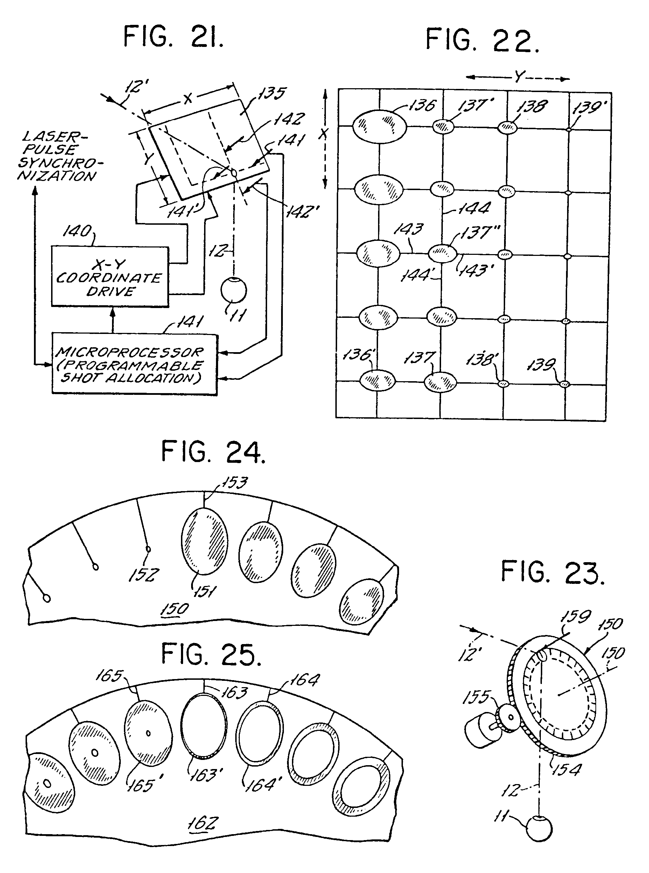

Figs. 21 to 26 are illustrative of a different aspect of the invention wherein the variously described sequences of spot shaping to achieve laser-ablated corneal-curvature change are produced by reflection techniques. And because the identification of parts in these figures corresponds with parts in Figs. 6, 7, 8, 9, 11 and 14, the same numbers are used, as applicable, in a 100-series.

In the embodiment of Figs. 21 and 22, a transparent plate 135, as of quartz, is characterized by a succession of elliptical reflection areas, oriented with their major axes parallel and respectively centered on each of the two-dimensionally (X-Y) indexible positions of plate 135. For each of the grid-like layouts of elliptical reflecting areas on plate 135, the size of the involved ellipse incrementally changes. Thus, for a first row of reflective ellipses beginning and ending with areas 136 and 136', respectively, the areas are progressively reducing; in the next-adjacent row, beginning and ending with areas 137 and 137', respectively, the reflecting ellipses continue their progressive reduction; in the third row, the progression continues to reduce from area 138 to area 138'; and the final row reduces still further from 139 to the smallest, 139'. Support for indexing displacement of plate 135 will be understood to position the reflective side thereof in inclined facing relation to the laser-output beam alignment 12', the inclination being preferably such that the major axis of each of the ellipses is at 45° to alignment 12' when the center of the particular ellipse has been indexed for intersection with the alignment 12'; at the same time, the minor axis of each ellipse is at 90° to alignment 12' when the center of the particular ellipse has been indexed for intersection with alignment 12', and the major/minor axis-span relation is $\sqrt{2}{:}1$. This preferred relation determines that for each ellipse-index position, the reflection 12 of the laser beam will be at 90° to the alignment 12' and that this reflection will be a circle of diameter equal to the minor-axis span of the involved ellipse. The X-Y coordinate index drive 140 and the microprocessor 141 perform as described for Figs. 6 and 7, and optically readable grid lines on plate 135 (between the reflective ellipses) enable optical-transducer pairs 141-141' and 142-142' to assure precise positioning of each reflecting ellipse, centered on axis 12', before firing the next laser pulse.

The automated running of the Fig. 21 device, in the full two-coordinate program of indexing plate 135, will be seen to deliver the greatest density of ablating energy in the central part of the total circular corneal area which is operated upon, with such density decreasing as a function of increasing radius from the optical axis of the eye. The curvature change is therefore of myopia-correcting nature.

The embodiment of Figs. 23 and 24 has its correspondence to Figs. 8 and 9, and thus the circumferentially distributed pattern of reflecting ellipses is on an indexible circular plate or disc 150, plate 150 being suitably transparent and of quartz. Preferably, the centers of all ellipses are on one geometrical circle about the index axis 150', and the index axis 150' is oriented to bisect the right-angle relation between laser axis 12 and the (reflected) projection axis 12' to the eye, axis 12' being aligned with the optical axis of the eye 11; also preferably, the major axis of each of the ellipses is oriented radially of the indexing center of plate 150, and, again, the major/minor axis relation of all ellipses is $\sqrt{2}{:}1$. The automated running of the rotary-indexed Fig. 23/24 arrangement will be seen to produce the same cornea-ablating result as the orthogonally indexed Fig. 21/22 arrangement, so that the result is again myopia-correcting.

The fragmentary showing of Fig. 25 illustrates that upon substitution of a different circular reflecting plate 162 (in place of plate 150 of Fig. 24), the microprocessor programming of rotary indexing and of laser pulsing will produce a hyperopia-correcting change in cornea curvature, of the nature shown in Fig. 10. The reflecting ellipses of Fig. 25 are in an angularly spaced succession of elliptical annuli of constant outer periphery; the succession ranges from the radially thinnest ellipse 163' at index location 163, to the radially thickest ellipse 165' at index location 165. In other words, the succession of reflecting ellipses of Fig. 25 accounts for annular projection of a constant outer diameter and of a varying inner diameter, throughout a single indexed rotation of plate 162, accounting for maximum ablating penetration of the cornea at the outer diameter, and progressively reduced ablating penetration as a function of decreasing radius about the optical axis of eye 11. For all ellipses, the major/minor axis ratio is $\sqrt{2}:1$, in view of the 45° incidence of the laser beam on each indexed elliptical reflector.

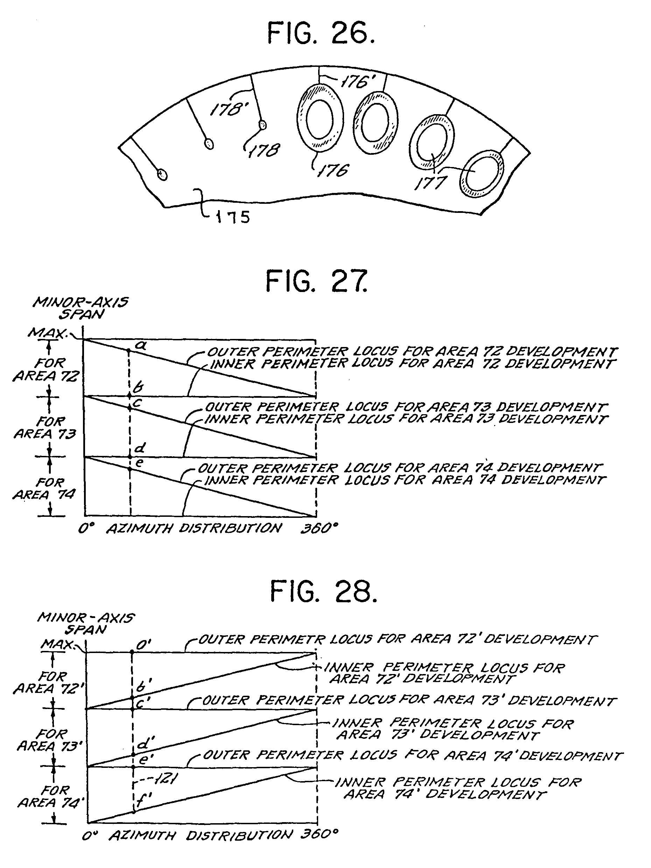

The arrangement of Fig. 26, taken with Figs. 12 and 13 is illustrative of the application of the reflection principles of Figs. 24 and 25 to corrective sculpture of the cornea to achieve a Fresnel-type distribution of the desired ultimate curvature, which, as for Fig. 15, can be either hyperopia-correcting or, as shown, myopia-correcting. To avoid too deep a cut of ablative penetration, the ultimate reduced-curvature surface, as at 31 in Fig. 4 (dashed line 71 in Fig. 13), is achieved in annular increments within the circular area bounded at 70, and the curvature 71 is produced at steps 72-73-74.

As shown in Fig. 26, a transparent plate 175 serves as a replacement for plate 150 in Fig. 23 and is provided with an angularly stepped progression of reflecting elliptical annuli, beginning with the largest and thickest elliptical annulus 176 at location 176', and proceeding clockwise to the next elliptical annulus of incrementally smaller size and thickness, based on an inner limiting ellipse 177 of constant size. For the three-step profile 72-73-74 shown, the reflecting elliptical annuli based on the same inner limiting ellipse 177, are distributed over a first 120° sector of disc 175, with the outer elliptical periphery progressively shrinking to a final radially thin ellipse (not shown); and the programmable means 57 (of Fig. 8) will be understood to function as a control for allocation of laser-pulse shots, using a programmed distribution of the first-sector elliptical reflectors, for ablative achievement of the curvature 71 within outer annulus 72. A similar succession of reflecting elliptical annuli will be understood to be similarly indexible over a second 120° sector (not shown) of disc 175, in establishing the curvature 71' within the intermediate annulus 73. And finally, the curvature 71" is established within the inner circular area 74 by programmed projection of laser shots on axis 12', through an indexibly available succession of progressively shrinking elliptical areas, beginning with an ellipse of largest minor-axis span (not shown, but equal to the diameter of the central circular area 74), and reducing throughout the third 120° sector to the smallest reflecting ellipse 178 at location 178', adjacent location 176' of the first sector.

A full rotation of disc 175, in the context of suitably programmed pulsed-laser delivery on alignment 12', thus creates the Fresnel steps 72-73-74 in succession. But it will be understood that by using the highly precise photo-reduction and metal-deposition techniques available from micro-circuit technology, each indexed step of a single disc (not shown) may be instrumental in the progressive formation of all annular components of a full Fresnel-type ablation pattern. To create reflecting elliptical patterns to achieve this result, Fig. 27 outlines the course of minor-axis size variation for all involved reflecting ellipses, for the case of myopia-correction, and Fig. 28 similarly outlines the course of minor-axis size variation for all involved reflecting ellipses, for the case of hyperopia correction.

In Fig. 27, it is seen that by dividing the full 360° angular extent of a given circular disc (utilizable in place of disc 150 in Fig. 23) into the desired number (n) of indexible steps at

$$\frac{360°}{n}$$

spacing, and by drawing an ordinate line (e.g., at 120) for each such increment of azimuthal distribution, intercepts (e.g., a-b-c-d-e, for location 120) are obtained for each of five loci, establishing the requisite minor-axis span for each of the involved plural reflecting ellipses at each particular index location. The result for Fig. 27 relationships is myopia-reducing because all outer perimeters (for areas 72-73-74) vary, while inner perimeters remain constant. On the other hand, the result for Fig. 28 relationships is hyperopia-reducing because all inner perimeters (for areas 72'-73'-74', not otherwise shown) vary, while outer perimeters remain constant, noting intercepts a'-b'-c'-d'-e'-f' for location 121.

All discussion thus far, for laser projection via indexed reflective areas, has been concerned with essentially spherical curvature correction, treating the myopic or the hyperopic situation, as the case may be. It should, however, also be apparent that similar principles are applicable to astigmatism correction, in which case the pattern of progressively indexed reflecting areas is rectangular, of progressively varying width, symmetrically developed on opposite sides of the central elongate axis of the most narrow rectangular pattern in the progression. The drawing of Fig. 16 may thus be considered illustrative of such a pattern development, wherein the indexible strip 80 is a transparent plate (as of quartz) and the series of rectangles 81 to 81' is reflecting and at equal centerline-to-centerline spacing, with indexing from one centerline (82) to the next, and with the laser-beam axis 12' directed at intersection with the central alignment 86 for each indexed position. It is realized that when strip 80 is supported on guide ring 84 and in an inclined plane as discussed for disc 150 in Fig. 23, the angular orientation of ring 84 (by setting adjustment at 85) will account for a range of width variation in rectangular spots incident at the eye, but the desired cummulative ablation can still be achieved at the eye for any and all selected angular orientations, by entering a suitable angularity correction into the microprocessor, the correction being a simple trigonometric function of orientation angle.

For the above-described reflective uses of the invention, it is to be understood that the individual patterns of reflection are operative upon a portion only of the laser-beam sectional area (on alignment 12'), and that, whether the reflective patterns are mounted to or formed upon a transparent plate (as of quartz) or are otherwise mounted, the portion of any given shot of laser-beam output that is not reflected will be further transmitted on essentially the alignment 12'. This further transmitted energy is not used for the surgery and may be trapped and dissipated by suitable means (not shown). Additionally, for some applications the reflector could be constituted by a variable aperture diaphragm characterized by a reflective side oriented to reflect the laser beam in a peripherally continuous annular area surrounding the diaphragm aperture.

## Claims

1. Apparatus for performing ophthalmological surgery by selective ablation of the anterior surface of the cornea with penetration into the stroma to achieve a volumetric removal of corneal tissue,

   said apparatus comprising laser means (13) for producing an output beam preferably in the ultraviolet portion of the electromagnetic spectrum and of an intensity which is limited per unit time to ablate but a fraction of a predetermined maximum ablation depth into the stroma, means (10,18,20) for directing said laser output beam onto the eye which is to be treated, and control means (16;46;57;89;141) for determining the irradiated flux density and exposure time of laser-beam impingement at the cornea whereby to effect a controlled sculpturing action upon the cornea such as to alter the optical properties thereof,

   and said apparatus further comprising adjustment means including a zoom lens (14;105) with a zoom drive (15;106) provided in the laser beam path for adjustably determining the size and/or shape of the laser beam area at impingement upon the cornea, and said control means (16;46;57;89;141) being operative upon said adjustment means for adjusting the laser beam area at impingement upon the cornea in a controlled manner in the course of a surgical treatment so that sculpturing action results from a controlled variation in the area of the cornea that is irradiated and a corresponding controlled variation in the cumulative ablative flux delivered to different areas of the cornea.

2. Apparatus according to claim 1 wherein said adjustment means (14,15) is arranged to vary the laser beam area within a predetermined maximum area and symmetrically with respect to a beam-projection axis which coincides with the optical axis of the eye.

3. Apparatus according to claim 1 or 2, in which an aperture of circular section is located between said laser and said zoom lens, and in which said zoom lens (14) images said aperture into a confined circular section of area which varies in accordance with the instantaneous setting of said zoom drive (15), whereby the surgical treatment may be myopia-correcting.

4. Apparatus according to claim 1 or 2, in which an elongate aperture of constant width is located between said laser and said zoom lens, and in which said zoom lens (105) images said aperture into a confined straight line of width which varies in accordance with the instantaneous setting of said zoom drive (106), whereby the surgical treatment may be corrective of astigmatism.

5. Apparatus according to claim 4, in which said zoom lens (105) is mounted for selective bodily rotation about the zoom-lens axis, whereby the angular orientation of said straight line may be set to accord with that of required astigmatism correction.

6. Apparatus for performing ophthalmological surgery by selective ablation of the anterior surface of the cornea with penetration into the stroma to achieve a volumetric removal of corneal tissue,

   said apparatus comprising laser means (13) for producing an output beam preferably in the ultraviolet portion of the electromagnetic spectrum and of an intensity which is limited per unit time to ablate but a fraction of a predetermined maximum ablation depth into the stroma, means (10,18,20) for directing said laser output beam onto the eye which is to be treated, and control means (16;46;57;89;141) for determining the irradiated flux density and exposure time of laser-beam impingement at the cornea whereby to effect a controlled sculpturing action upon the cornea such as to alter the optical properties thereof,

   and said apparatus further comprising variable area masking means (35;50;62;75;80;101, 102,104) provided in the laser beam path for adjustably determining the size and/or shape of the laser beam area at impingement upon the cornea and actuating means (40;55;88;100) for varying the area of the opening of the masking means, and said control means (16;46;57;89;141) being operative upon said actuating means for adjusting the laser beam area at impingement upon the cornea in a controlled manner in the course of a surgical treatment so that sculpturing action results from a controlled variation in the area of the cornea that is irradiated and a corresponding controlled variation in the cumulative ablative flux delivered to different areas of the cornea.

7. Apparatus according to claim 6 wherein said masking means comprises a variable aperture diaphragm.

8. Apparatus according to claim 6 or 7, in which said masking means (35;50) is operative to provide cornea exposure at a maximum area and at a given plurality of similarly shaped but lesser areas, said areas being circular, whereby the surgical treatment may be myopia-correcting.

9. Apparatus according to claim 6 or 7, in which said masking means (80;101,102,104) is operative to provide cornea exposure at a maximum area and at a given plurality of similarly shaped but lesser areas, said areas being elongate rectangular and of varying width, whereby the surgical treatment may be astigmatism-correcting.

10. Apparatus according to claim 9, wherein the orientation of the elongate direction of said areas is adjustable.

11. Apparatus according to claim 6, in which said masking means (62) is operative to provide cornea exposure a a maximum area and at a given plurality of similarly shaped but lesser areas, said areas being circularly annular and being defined by a constant outer diameter and a varying inner diameter, whereby the surgical treatment may be hyperopia-correcting.

12. Apparatus according to claim 6, in which said masking means (75) is operative to provide cornea exposure at a maximum area and at a given plurality of similarly shaped but lesser areas, said areas being circularly annular and being defined by a constant inner diameter and by a varying outer diameter which is intermediate said inner diameter and the outer diameter of said maximum area, whereby the surgical treatment may be myopia-correcting in a sculpted Fresnel annulus defined by said inner and outer diameters.

13. Apparatus according to claim 6, in which said masking means is operative to provide cornea exposure at a maximum area and at a given plurality of similarly shaped but lesser areas, said areas being circularly annular, being defined by a constant outer diameter and by an inner diameter which varies to a fixed minimum inner diameter, whereby the surgical treatment may be hyperopia-correcting in a sculpted Fresnel annulus defined by said constant outer diameter and by said fixed minimum inner diameter.

14. Apparatus according to claim 6 or any of claims 8 to 13 as dependent upon claim 6, in which said masking means (35;50;62;75;80) comprises an opaque plate having a succession of windows which are transparent to laser-beam transmission therethrough and of progressively changing area, and said actuating means (40;55;88) is microprocessor-controlled for indexing said windows into successive alignment with the axis of the laser beam.

15. Apparatus for performing ophthalmological surgery by selective ablation of the anterior surface of the cornea with penetration into the stroma to achieve a volumetric removal of corneal tissue, said apparatus comprising laser means (13) for producing an output beam preferably in the ultraviolet portion of the electromagnetic spectrum and of an intensity which is limited per unit time to ablate but a fraction of a predetermined maximum ablation depth into the stroma, means (10,18,20) for directing said laser output beam onto the eye which is to be treated, and control means (16;46;57;89;141) for determining the irradiated flux density and exposure time of laser-beam impingement at the cornea whereby to effect a controlled sculpturing action upon the cornea such as to alter the optical properties thereof, and said apparatus further comprising reflector means (135;150;162;175) provided in the laser beam path for variably limiting the area of the laser beam at impingement upon the cornea and actuating means (140;155) for varying the reflector area of said reflector means, and said control means (16;46;57;89;141) being operative upon said actuating means for adjusting the laser beam area at impingement upon the cornea in a controlled manner in the course of a surgical treatment so that sculpturing action results from a controlled variation in the area of the cornea that is irradiated and a corresponding controlled variation in the cumulative ablative flux delivered to different areas of the cornea.

16. Apparatus according to claim 15, in which said reflector means (135;150) is operative to provide cornea exposure at a central circular area and at a given plurality of similarly shaped but greater areas, said areas being concentric, whereby the surgical treatment may be myopia-correcting.

17. Apparatus according to claim 15, in which said reflector means (162) is operative to provide cornea exposure at a maximum area and at a given plurality of similarly shaped but lesser areas, said areas being circularly annular and being defined by a constant outer diameter and a varying inner diameter whereby the surgical treatment may be hyperopia-correcting.

18. Apparatus according to claim 15, in which said reflector means is operative to provide cornea exposure at a narrow elongate rectangular area centered on the optical axis of the eye and spanning a maximum area, plural said reflector means being further operative to provide cornea exposure at similarly shaped but greater areas, said areas being elongate rectangular and of varying width which is symmetrical about the elongation direction of said narrow area, whereby the surgical treatment may be astigmatism-correcting.

19. Apparatus according to claim 18, wherein orientation of the elongate direction of said areas is adjustable.

20. Apparatus according to claim 15, in which said reflector means (175) is operative to provide cornea exposure at a maximum area and at a given plurality of similarly shaped but lesser areas, said areas being circularly annular and being defined by a constant inner diameter and by a varying outer diameter which is intermediate said inner diameter and the outer diameter of said maximum area, whereby the surgical treatment may be myopia-correcting in a sculpted Fresnel annulus defined by said inner and outer diameters.

21. Apparatus according to claim 15, in which said reflector means is operative to provide cornea exposure at a maximum area and at a given plurality of similarly shaped but lesser areas, said areas being circularly annular, being defined by a constant outer diameter and by an inner diameter which varies to a fixed minimum inner diameter, whereby the surgical treatment may be hyperopia-correcting in a sculpted Fresnel annulus defined by said constant outer diameter and by said fixed minimum inner diameter.

22. Apparatus according to any of claims 15 to 21, in which said reflector means (135;150;162;175) comprises a transparent plate having a succession of reflecting elements on a surface thereof, said reflecting elements being of progressively changing area, and said actuating means (140;155) is microprocessor-controlled for indexing said reflecting elements into successive alignment with the axis of the laser beam.

23. Apparatus according to claim 15, in which said reflector means is a variable-aperture diaphragm characterised by a reflective side oriented to reflect the laser beam in a peripherally continuous annular area surrounding the diaphragm aperture.

24. Apparatus according to any of claims 15 to 22, in which laser-beam incidence upon said reflector means is at 45 degrees, and in which the reflector area is elliptical with a major-axis to minor-axis ratio of $\sqrt{2}:1$, the laser-beam incidence being centered on the ellipse and at 45 degrees to the major-axis thereof.

25. A method, excluding the application of this method on the living human and/or animal body, of corneal sculpture by selective ablation of the corneal surface with penetration into the stroma so as to achieve a volumetric removal of corneal tissue, said method comprising:

utilizing laser means (13) for producing an output beam preferably in the ultraviolet portion of the electromagnetic spectrum and of an intensity which is limited per unit time to ablate but a fraction of a predetermined maximum ablation depth into the stroma;

directing said laser output beam through a zoom lens (14;105) provided in the beam path onto the cornea which is to be sculpted; and

controlling the irradiated flux density and exposure time of laser-beam impingement at the cornea whereby to effect a controlled sculpting action upon the cornea such as to alter the optical properties thereof, such control being effected by adjusting the zoom lens (14;105) so as to adjust the size of the laser beam area at impingement upon the cornea so that sculpturing action results from a controlled variation in the area of the cornea that is irradiated and a corresponding controlled variation in the cumulative ablative flux delivered to different areas of the cornea.

26. A method, excluding the application of this method on the living human and/or animal body, of corneal sculpture by selective ablation of the corneal surface with penetration into the stroma so as to achieve a volumetric removal of corneal tissue, said method comprising:

utilizing laser means (13) for producing an output beam preferably in the ultraviolet portion of the electromagnetic spectrum and of an intensity which is limited per unit time to ablate but a fraction of a predetermined maximum ablation depth into the stroma;

directing said laser output beam onto the cornea which is to be sculpted; and

controlling the irradiated flux density and exposure time of laser-beam impingement at the cornea whereby to effect a controlled sculpting action upon the cornea such as to alter the optical properties thereof, such control being effected by adjusting the size and/shape of the laser beam area at impingement upon the cornea so that sculpturing action results from a controlled variation in the area of the cornea that is irradiated and a corresponding controlled variation in the cumulative ablative flux delivered to different areas of the cornea;

the adjustment of the laser beam area at corneal impingement being effected by selective use of variable-area beam-masking means (35;50;62;75;80;101,102,104) and by varying the area of the opening of the masking means in order to define a plurality of given different sizes and/or shapes of laser beam area at corneal impingement.

27. A method, excluding the application of this method on the living human and/or animal body, of corneal sculpture by selective ablation of the corneal surface with penetration into the stroma so as to achieve a volumetric removal of corneal tissue, said method comprising:

utilizing laser means (13) for producing an output beam preferably in the ultraviolet portion of the electromagnetic spectrum and of an intensity which is limited per unit time to ablate but a fraction of a predetermined maximum ablation depth into the stroma;

directing said laser output beam onto the cornea which is to be sculpted; and

controlling the irradiated flux density and exposure time of laser-beam impingement at the cornea whereby to effect a controlled sculpting action upon the cornea such as to alter the optical properties thereof, such control being effected by adjusting the size and/or shape of the laser beam area at impingement upon the cornea so that sculpturing action results from a controlled variation in the area of the cornea that is irradiated and a corresponding controlled variation in the cumulative ablative flux delivered to different areas of the cornea;

the adjustment of the laser beam area at corneal impingement being effected by selective use of beam reflection means (135;150;162;175) defining a plurality of given different sizes and/or shapes of laser beam area at corneal impingement.

28. A method according to any of claims 25 to 27 wherein sculpturing action is effected within a predetermined central area of the cornea and with the cornea being subjected to the greatest density of laser beam exposure centrally of said central area and to an exposure density which decreases with increasing radius to the outer regions of said central area.

29. A method according to claim 26 or 27 wherein sculpturing action is effected within a predetermined central area of the cornea and with the cornea being subjected to the least density of laser beam exposure centrally of said central area and to an exposure density which increases with increasing radius to the outer regions of said central area.

30. A method according to any of claims 25 to 27 wherein the laser beam has an elongate rectangular shape at corneal impingement and sculpturing action is effected with the axis of elongation of the beam fixed in a determined orientation and by varying the width of the rectangular beam transverse to its said axis so that an elongate central region of the cornea is subjected to the greatest density of laser beam exposure and to an exposure density which deceases with increasing lateral offset on either side of said elongate central region.

31. A method according to claim 30 wherein the fixing of the axis of elongation of the laser beam is effected automatically as a function of diagnosis of the optical characteristics of the cornea that is to be sculptured.

32. A method according to any of claims 25 to 31 wherein sculpturing action is effected in a series of operations so as to achieve a Fresnel recharacterization of the corneal surface.

**Patentansprüche**

1. Gerät zum Ausführen von augenärztlicher Chirurgie durch selektives Abtragen der Vorderfläche der Augenhornhaut unter Eindringen in das Bindegewebe zum Erzielen einer Volumensbeseitigung von Hornhautgewebe,

wobei das Gerät eine Laservorrichtung (17) zum Erzeugen eines Ausgangsstrahls vorzugsweise im Ultraviolettbereich des elektromagnetischen Spektrums und mit einer Intensität, die je Zeiteinheit zum Abtragen von nur einem Bruchteil einer vorbestimmten maximalen Abtragetiefe in das Bindegewebe begrenzt ist, eine Vorrichtung (10, 18, 20) zum Richten des Laserausgangsstrahls auf das zu behandelnde Auge und eine Steuereinrichtung (16; 46; 57; 89; 141) zum Bestimmen der Bestrahlungsflußdichte und der Belichtungszeit der Laserstrahlbeaufschlagung an der Hornhaut enthält, um dadurch eine gesteuerte Skulpturierung auf der Hornhaut derart auszuführen, daß sich deren optische Eigenschaften ändern,

und wobei das Gerät ferner eine in dem Laserstrahlweg angebrachte Einstellvorrichtung, die eine Zoomlinse (14; 105) mit einem Zoomstellantrieb (15; 106) enthält, zum einstellbaren Bestimmen der Größe und/oder Form der Laserstrahlfläche bei dem Auftreffen auf die Hornhaut aufweist und die Steuereinrichtung (16; 46; 57; 89; 141) auf die Einstellvorrichtung zum Einstellen der Laserstrahlfläche bei dem Auftreffen auf die Hornhaut auf gesteuerte Weise im Ablauf einer chirurgischen Behandlung derart einwirkt, daß sich die Skulpturierung aus einer gesteuerten Änderung der bestrahlten Fläche der Hornhaut und einer entsprechenden gesteuerten Änderung der an die verschiedenen Flächen der Hornhaut abgegebenen Summen-Abtrageströmung ergibt.

2. Gerät nach Anspruch 1, in dem die Einstellvorrichtung (14, 15) zum Ändern der Laserstrahlfläche innerhalb einer vorbestimmten Maximalfläche und symmetrisch in bezug auf eine Strahlprojektionsachse gestaltet ist, die mit der optischen Achse des Auges übereinstimmt.

3. Gerät nach Anspruch 1 oder 2, in dem zwischen dem Laser und der Zoomlinse eine Blende mit kreisförmigem Querschnitt angeordnet ist und in dem die Zoomlinse (14) die Blende als einen verengten kreisförmigen Flächenabschnitt abbildet, der sich entsprechend der momentanen Stellung des Zoomstellantriebs (15) ändert, wodurch mit der chirurgischen Behandlung eine Kurzsichtigkeitskorrektur ermöglicht ist.

4. Gerät nach Anspruch 1 oder 2, in dem zwischen dem Laser und der Zoomlinse eine Schlitzblende mit konstanter Breite angeordnet ist und in dem die Zoomlinse (105) die Blende als eine verengte gerade Linie mit einer Breite abbildet, die sich entsprechend der momentanen Stellung des Zoomstellantriebs (106) ändert, wodurch mit der chirurgischen Behandlung eine Astigmatismuskorrektur ermöglicht ist.

5. Gerät nach Anspruch 4, in dem die Zoomlinse (105) zu einer selektiven körperlichen Drehung um die Zoomlinsenachse eingebaut ist, wodurch die Winkelausrichtung der geraden Linie zum Übereinstimmen mit derjenigen für die geforderte Astigmatismuskorrektur einstellbar ist.

6. Gerät zum Ausführen von augenärztlicher Chirurgie durch selektives Abtragen der Vorderfläche der Augenhornhaut unter Eindringen in das Bindegewebe zum Erzielen einer Volumensbeseitigung von Hornhautgewebe,

wobei das Gerät eine Laservorrichtung (17) zum Erzeugen eines Ausgangsstrahls vorzugsweise im Ultraviolettbereich des elektromagnetischen Spektrums und mit einer Intensität, die je nach Zeiteinheit zum Abtragen von nur einem Bruchteil einer vorbestimmten maximalen Abtragetiefe in das Bindegewebe begrenzt ist, eine Vorrichtung (10, 18, 20) zum Richten des Laserausgangsstrahls auf das zu behandelnde Auge und eine Steuereinrichtung (16; 46; 57; 89; 141) zum Bestimmen der Bestrahlungsflußdichte und der Belichtungszeit der Laserstrahlbeaufschlagung an der Hornhaut enthält, um dadurch eine gesteuerte Skulpturierung auf der Hornhaut derart auszuführen, daß sich deren optische Eigenschaften ändern,

und wobei das Gerät ferner eine in dem Laserstrahlweg angebrachte Maskiervorrichtung (35; 50; 62; 75; 80; 101, 102, 104) mit veränderbarer Fläche zum einstellbaren Bestimmen der Größe und/oder Form der Laserstrahlfläche bei dem Auftreffen auf die Hornhaut und eine Stellvorrichtung (40; 55; 88; 100) zum Verändern der Öffnungsfläche der Maskiervorrichtung aufweist und die Steuereinrichtung (16; 46; 57; 89; 141) auf die Einstellvorrichtung zum Einstellen der Laserstrahlfläche bei dem Auftreffen auf die Hornhaut auf gesteuerte Weise im Ablauf einer chirurgischen Behandlung derart einwirkt, daß sich die Skulpturierung aus einer gesteuerten Änderung der bestrahlten Fläche der Hornhaut und einer entsprechenden gesteuerten Änderung der an die verschiedenen Flächen der Hornhaut abgegebenen Summen-Abtrageströmung ergibt.

7. Gerät nach Anspruch 6, in dem die Maskiervorrichtung eine Blende mit veränderbarer Öffnung enthält.

8. Gerät nach Anspruch 6 oder 7, in dem die Maskiervorrichtung (35; 50) zum Belichten der Hornhaut auf einer maximalen Fläche und auf einer bestimmten Vielzahl von gleichartig geformten, aber kleineren Flächen betreibbar ist, die kreisförmig sind, wodurch mit der chirurgischen Behandlung eine Kurzsichtigkeitskorrektur ermöglicht ist.

9. Gerät nach Anspruch 6 oder 7, in dem die Maskiervorrichtung (80; 101, 102, 104) zum Belichten der Hornhaut auf einer maximalen Fläche und auf einer bestimmten Vielzahl von gleichartig geformten, aber kleineren Flächen betreibbar ist, die langgestreckt rechteckförmig und von veränderbarer Breite sind, wodurch mit der chirurgischen Behandlung eine Astigmatismuskorrektur ermöglicht ist.

10. Gerät nach Anspruch 9, in dem die Ausrichtung der Längsrichtung der Flächen einstellbar ist.

11. Gerät nach Anspruch 6, in dem die Maskiervorrichtung (62) zum Belichten der Hornhaut auf einer maximalen Fläche und auf einer bestimmten Vielzahl von gleichartig geformten, aber kleineren Flächen betreibbar ist, die kreisringförmig sind und die durch einen konstanten Außendurchmesser und einen veränderbaren Innendurchmesser begrenzt sind, wodurch mit der chirurgischen Behandlung eine Weitsichtigkeitskorrektur ermöglicht ist.

12. Gerät nach Anspruch 6, in dem die Maskiervorrichtung (75) zum Belichten der Hornhaut auf einer maximalen Fläche und auf einer bestimmten Vielzahl von gleichartig geformten, aber kleineren Flächen betreibbar ist, die kreisringförmig sind und die durch einen konstanten Innendurchmesser und einen veränderbaren Außendurchmesser begrenzt sind, der zwischen dem Innendurchmesser und dem Außendurchmesser der maximalen Fläche liegt, wodurch mit der chirurgischen Behandlung eine Kurzsichtigkeitskorrektur in einem skulpturierten Fresnel-Ring ermöglicht ist, der durch den Innendurchmesser und den Außendurchmesser begrenzt ist.

13. Gerät nach Anspruch 6, in dem die Maskiervorrichtung zum Belichten der Hornhaut auf einer maximalen Fläche und auf einer bestimmten Vielzahl von gleichartig geformten, aber kleineren Flächen betreibbar ist, die kreisringförmig sind und die durch einen konstanten Außendurchmesser und einen Innendurchmesser begrenzt sind, der sich bis zu einem festgelegten minimalen Innendurchmesser ändert, wodurch mit der chirurgischen Behandlung eine Weitsichtigkeitskorrektur in einem skulpturierten Fresnel-Ring ermöglicht ist, der durch den konstanten Außendurchmesser und durch den festgelegten minimalen Innendurchmesser begrenzt ist.

14. Gerät nach Anspruch 6 oder einem auf den Anspruch 6 rückbezogenen Anspruch der Ansprüche 8 bis 13, in dem die Maskiervorrichtung (35; 50; 62; 75; 80) eine undurchlässige Platte mit einer Reihe von Fenstern enthält, die für den Laserstrahl durchlässig sind und fortschreitend geänderte Flächen haben, und die Stellvorrichtung (40; 55; 88) von einem Mikroprozessor zum Fortschalten der Fenster in aufeinanderfolgende Ausfluchtung mit der Achse des Laserstrahls gesteuert ist.

15. Gerät zum Ausführen von augenärztlicher Chirurgie durch selektives Abtragen der Vorderfläche der Augenhornhaut unter Eindringen in das Bindegewebe zum Erzielen einer Volumensbeseitigung von Hornhautgewebe,

wobei das Gerät eine Laservorrichtung (17) zum Erzeugen eines Ausgangsstrahls vorzugsweise im Ultraviolettbereich des elektromagnetischen Spektrums und mit einer Intensität, die je nach Zeiteinheit zum Abtragen von nur einem Bruchteil einer vorbestimmten maximalen Abtragetiefe in das Bindegewebe begrenzt ist, eine Vorrichtung (10, 18, 20) zum Richten des Laserausgangsstrahls auf das zu behandelnde Auge und eine Steuereinrichtung (16; 46; 57; 89; 141) zum Bestimmen der Bestrahlungsflußdichte und der Belichtungszeit der Laserstrahlbeaufschlagung an der Hornhaut enthält, um dadurch eine gesteuerte Skulpturierung auf der Hornhaut derart auszuführen, daß sich deren optische Eigenschaften ändern,

und wobei das Gerät ferner eine in dem Laserstrahlweg angebrachte Spiegelvorrichtung (135; 150; 162; 175) zum veränderbaren Begrenzen der Fläche des Laserstrahls bei dem Auftreffen auf die Hornhaut und eine Stellvorrichtung (140; 155) zum Verändern der Spiegelfläche der Spiegelvorrichtung aufweist und die Steuereinrichtung (16; 46; 57; 89; 141) auf die Stellvorrichtung zum Einstellen der Laserstrahlfläche bei dem Auftreffen auf die Hornhaut auf gesteuerte Weise im Ablauf einer chirurgischen Behandlung derart einwirkt, daß sich die Skulpturierung aus einer gesteuerten Änderung der bestrahlten Fläche der Hornhaut und einer entsprechenden gesteuerten Änderung der an die verschiedenen Flächen der Hornhaut abgegebenen Summen-Abtrageströmung ergibt.

16. Gerät nach Anspruch 15, in dem die Spiegelvorrichtung (135; 150) zum Belichten der Hornhaut auf einer mittigen kreisförmigen Fläche und auf einer bestimmten Vielzahl von gleichartig geformten, aber größeren Flächen betreibbar ist, die konzentrisch sind, wodurch mit der chirurgischen Behandlung eine Kurzsichtigkeitskorrektur ermöglicht ist.

17. Gerät nach Anspruch 15, in dem die Spiegelvorrichtung (162) zum Belichten der Hornhaut auf einer maximalen Fläche und auf einer bestimmten Vielzahl von gleichartig geformten, aber kleineren Flächen betreibbar ist, die kreisringförmig sind und durch einen konstanten Außendurchmesser und einen sich ändernden Innendurchmesser begrenzt sind, wodurch mit der chirurgischen Behandlung eine Weitsichtigkeitskorrektur ermöglicht ist.

18. Gerät nach Anspruch 15, in dem die Spiegelvorrichtung zum Belichten der Hornhaut auf einer schmalen langgestreckten Rechteckfläche betreibbar ist, die auf der optischen Achse des Auges zentriert ist und die eine maximale Fläche umfaßt, wobei ferner mehrere Spiegelvorrichtungen zum Belichten der Hornhaut an gleichartig geformten, aber größeren Flächen betreibbar sind, die langgestreckt rechteckig und von veränderter Breite sind, welche um die Längsrichtung der schmalen Fläche symmetrisch ist, wodurch mit der chirurgischen Behandlung eine Astigmatismuskorrektur ermöglicht ist.

19. Gerät nach Anspruch 18, in dem die Ausrichtung der Längsrichtung der Flächen einstellbar ist.

20. Gerät nach Anspruch 15, in dem die Spiegelvorrichtung (175) zum Belichten der Hornhaut auf einer maximalen Fläche und auf einer bestimmten Vielzahl von gleichartig geformten, aber kleineren Flächen betreibbar ist, die kreisringförmig sind und von einem konstanten Innendurchmesser und einem sich ändernden Außendurchmesser begrenzt sind, der zwischen dem Innendurchmesser und dem Außendurchmesser der maximalen Fläche liegt, wodurch mit der chirurgischen Behandlung eine Kurzsichtigkeitskorrektur in einem skulpturierten Fresnel-Ring ermöglicht ist, der durch den Innendurchmesser und den Außendurchmesser begrenzt ist.

21. Gerät nach Anspruch 15, in dem die Spiegelvorrichtung zum Belichten der Hornhaut auf einer maximalen Fläche und auf einer bestimmten Vielzahl von gleichartig geformten, aber kleineren Flächen betreibbar ist, die kreisringförmig sind und die durch einen konstanten Außendurchmesser und einen Innendurchmesser begrenzt sind, der sich bis zu einem festgelegten minimalen Innendurchmesser ändert, wodurch mit der chirurgischen Behandlung eine Weitsichtigkeitskorrektur in einem skulpturierten Fresnel-Ring ermöglicht ist, der durch den konstanten Außendurchmesser und durch den festgelegten minimalen Innendurchmesser begrenzt ist.

22. Gerät nach einem der Ansprüche 15 bis 21, in dem die Spiegelvorrichtung (135; 150; 162; 175) eine durchlässige Platte mit einer Reihe von Spiegelelementen an einer Oberfläche derselben enthält, die fortschreitend veränderte Flächen haben, und die Stellvorrichtung (140; 155) von einem Mikroprozessor zum Fortschalten der Spiegelelemente in aufeinanderfolgende Ausfluchtung mit der Achse des Laserstrahls gesteuert ist.

23. Gerät nach Anspruch 15, in dem die Spiegelvorrichtung eine Blende mit veränderbarer Öffnung ist, die durch eine zum Reflektieren des Laserstrahls ausgerichtete reflektierende Seite in einem die Blendenöffnung umgebenden, auf dem Umfang durchgehenden ringförmigen Bereich gekennzeichnet ist.

24. Gerät nach einem der Ansprüche 15 bis 22, in dem der Laserstrahl unter 45° auf die Spiegelvorrichtung fällt und in dem die Spiegelfläche elliptisch mit einem Hauptachse:Nebenachse-Verhältnis von 2:1 ist, wobei der Laserstrahl auf die Ellipse zentriert und unter 45° zu deren Hauptachse einfällt.

25. Verfahren, unter Ausschluß der Anwendung dieses Verfahrens an dem lebenden menschlichen und/oder tierischen Körper, zur Augenhornhaut-Skulpturierung durch selektives Abtragen der Hornhautoberfläche unter Eindringen in das Bindegewebe in der Weise, daß eine Volumensbeseitigung von Hornhautgewebe erzielt wird, wobei das Verfahren umfaßt:

das Anwenden einer Laservorrichtung (13) zum Erzeugen eines Ausgangsstrahls vorzugsweise in dem Ultraviolettbereich des elektromagnetischen Spektrums und mit einer Intensität, die je Zeiteinheit zum Abtragen nur eines Bruchteils einer vorbestimmten maximalen Abtragungstiefe in das Bindegewebe begrenzt ist,

das Richten des Laserausgangsstrahls über eine im Strahlweg angebrachte Zoomlinse (14; 105) auf die zu skulpturisierende Hornhaut und

das Steuern der Bestrahlungsflußdichte und der Belichtungszeit der Laserstrahlenbeaufschlagung an der Hornhaut, wodurch eine gesteuerte Skulpturierung an der Hornhaut in der Weise bewirkt wird, daß sich deren optische Eigenschaften verändern, wobei die Steuerung durch Einstellen der Zoomlinse (14; 105) zum Einstellen der Größe des Laserstrahlbereichs bei dem Auftreffen auf die Hornhaut in der Weise herbeigeführt wird, daß sich die Skulpturierung aus einer gesteuerten Änderung der bestrahlten Fläche der Hornhaut und einer entsprechend gesteuerten Änderung der auf die verschiedenen Flächen der Hornhaut aufgebrachten Summen-Abtragströmung ergibt.

26. Verfahren, unter Ausschluß der Anwendung dieses Verfahrens an dem lebenden menschlichen und/oder tierischen Körper, zur Augenhornhaut-Skulpturierung durch selektives Abtragen der Hornhautoberfläche unter Eindringen in das Bindegewebe in der Weise, daß eine Volumensbeseitigung von Hornhautgewebe erzielt wird, wobei das Verfahren umfaßt:

das Anwenden einer Laservorrichtung (13) zum Erzeugen eines Ausgangsstrahls vorzugsweise in dem Ultraviolettbereich des elektromagnetischen Spektrums und mit einer Intensität, die je Zeiteinheit zum Abtragen nur eines Bruchteils einer vorbestimmten maximalen Abtragungstiefe in das Bindegewebe begrenzt ist,

das Richten des Laserausgangsstrahls auf die zu skulpturisierende Hornhaut und

das Steuern der Bestrahlungsflußdichte und der Belichtungszeit der Laserstrahlenbeaufschlagung an der Hornhaut, wodurch eine gesteuerte Skulpturierung an der Hornhaut in der Weise bewirkt wird, daß sich deren optische Eigenschaften verändern, wobei die Steuerung durch das Einstellen der Größe und/oder Form des Laserstrahlbereichs bei dem Auftreffen auf die Hornhaut in der Weise herbeigeführt wird, daß sich die Skulpturierung aus einer gesteuerten Änderung der bestrahlten Fläche der Hornhaut und einer entsprechend gesteuerten Änderung der auf die verschiedenen Flächen der Hornhaut aufgebrachten Summen-Abtragströmung ergibt,

wobei die Einstellung des Laserstrahlbereichs bei dem Auftreffen auf die Hornhaut einer Strahl-Maskiervorrichtung (35; 50; 62; 75; 80; 101, 102, 104) mit veränderbarer Fläche und durch Verändern der Öffnungsfläche der Maskiervorrichtung erfolgt, um eine Vielzahl von bestimmten unterschiedlichen Größen und/oder Formen des Laserstrahlbereichs bei dem Auftreffen auf die Hornhaut zu bestimmen.

27. Verfahren, unter Ausschluß der Anwendung dieses Verfahrens an dem lebenden menschlichen und/oder tierischen Körper, zur Augenhornhaut-Skulpturierung durch selektives Abtragen der Hornhautoberfläche unter Eindringen in das Bindegewebe in der Weise, daß eine Volumensbeseitigung von Hornhautgewebe erzielt wird, wobei das Verfahren umfaßt:

das Anwenden einer Laservorrichtung (13) zum Erzeugen eines Ausgangsstrahls vorzugsweise in dem Ultraviolettbereich des elektromagnetischen Spektrums und mit einer Intensität, die je Zeiteinheit zum Abtragen nur eines Bruchteils einer vorbestimmten maximalen Abtragungstiefe in das Bindegewebe begrenzt ist,

das Richten des Laserausgangsstrahls auf die zu skulpturisierende Hornhaut und

das Steuern der Bestrahlungsflußdichte und der Belichtungszeit der Laserstrahlenbeaufschlagung an der Hornhaut, wodurch eine gesteuerte Skulpturierung an der Hornhaut in der Weise bewirkt wird, daß sich deren optische Eigenschaften verändern, wobei die Steuerung durch das Einstellen der Größe und/oder Form des Laserstrahlbereichs bei dem Auftreffen auf die Hornhaut in der Weise herbeigeführt wird, daß sich die Skulpturierung aus einer gesteuerten Änderung der bestrahlten Fläche der Hornhaut und einer entsprechend gesteuerten Änderung der auf die verschiedenen Flächen der Hornhaut aufgebrachten Summen-Abtragströmung ergibt,

wobei die Einstellung der Laserstrahlfläche bei dem Auftreffen auf die Hornhaut durch das selektive Einsetzen einer Strahlreflexionsvorrichtung (135; 150; 162; 175) herbeigeführt wird, welche eine Vielzahl von bestimmten unterschiedlichen Größen und/oder Formen der Laserstrahlfläche bei dem Auftreffen auf die Hornhaut bestimmt.

28. Verfahren nach einem der Ansprüche 25 bis 27, bei dem die Skulpturierung innerhalb eines vorbestimmten Mittenbereichs der Hornhaut herbeigeführt wird, wobei die Hornhaut in der Mitte des Mittenbereichs der größten Dichte der Laserstrahlbelichtung und einer Belichtungsdichte ausgesetzt wird, die zu den Außenbereichen des Mittenebreichs hin mit zunehmendem Radius abnimmt.

29. Verfahren nach Anspruch 26 oder 27, bei dem die Skulpturierung innerhalb eines vorbestimmten Mittelbereichs der Hornhaut vorgenommen wird, wobei die Hornhaut in der Mitte des Mittelbereichs der geringsten Dichte der Laserstrahlbelichtung und einer Belichtungsdichte ausgesetzt wird, die zu den Außenbereichen des Mittelbereichs hin mit zunehmendem Radius zunimmt.

30. Verfahren nach einem der Ansprüche 25 bis 27, bei dem der Laserstrahl bei dem Auftreffen auf die Hornhaut eine langgestreckte rechteckige Form hat und die Skulpturierung unter Festlegen der Längsachse des Strahls in eine bestimmte Ausrichtung und durch Verändern der Breite des rechteckigen Strahls quer zu dessen Achse in der Weise herbeigeführt wird, daß ein langgestreckter Mittenbereich der Hornhaut der Laserstrahlbelichtung in der größten Dichte und einer Belichtungsdichte ausgesetzt wird, die mit zunehmender seitlicher Versetzung beiderseits des langgestreckten Mittenbereichs abnimmt.

31. Verfahren nach Anspruch 30, bei dem das Festlegen der Längsachse des Laserstrahls auf automatische Weise als eine Funktion einer Diagnose der optischen Eigenschaften der zu verformenden Hornhaut vorgenommen wird.

32. Verfahren nach einem der Ansprüche 25 bis 31, bei dem die Skulpturierung in einer Folge von Vorgängen in der Weise herbeigeführt wird, daß eine Fresnel-Rückbildung der Hornhautoberfläche erzielt wird.

## Revendications

1. Appareil pour effectuer de la chirurgie ophtalmologique par ablation sélective de la surface antérieure de la cornée, avec pénétration dans le stroma, pour réaliser un retrait volumétrique de tissu cornéen,
   ledit appareil comprenant :
   - un moyen formant laser (13) pour produire un faisceau de sortie, de préférence dans la région de l'ultraviolet du spectre électromagnétique, et d'une intensité qui est limitée par unité de temps pour n'enlever qu'une fraction d'une profondeur d'ablation maximale prédéterminée dans le stroma ;
   - un moyen (10, 18, 20) pour diriger ledit faisceau de sortie du laser sur l'oeil qui doit être traité ; et
   - un moyen de commande (16 ; 46 ; 57 ; 89 ; 141) pour déterminer la densité de flux irradié et le temps d'exposition de l'impact du faisceau laser sur la cornée, pour effectuer ainsi une action de sculpture contrôlée sur la cornée de façon à en modifier les propriétés optiques ; et
   ledit appareil comprenant en outre :
   - un moyen d'ajustement incluant une lentille à focale variable (14 ; 105) avec une commande de focale variable (15 ; 106), disposé sur la trajectoire du faisceau laser pour déterminer de façon ajustable la dimension et/ou la forme de la surface du faisceau laser au niveau de l'impact sur la cornée ; et
   - ledit moyen de commande (16 ; 46 ; 57 ; 89 ; 141) agissant sur ledit moyen d'ajustement pour ajuster la surface du faisceau laser au niveau de l'impact sur la cornée d'une manière contrôlée au cours d'un traitement chirurgical, de telle sorte que l'action de sculpture résulte d'une variation contrôlée de la surface de la cornée qui est irradiée et d'une variation contrôlée correspondante du flux d'ablation cumulé adressé à différentes zones de la cornée.

2. Appareil selon la revendication 1, dans lequel ledit moyen d'ajustement (14, 15) est prévu pour faire varier la surface du faisceau laser à l'intérieur d'une surface maximale prédéterminée et symétriquement par rapport à un axe de projection du faisceau qui coïncide avec l'axe optique de l'oeil.

3. Appareil selon la revendication 1 ou 2, dans lequel une ouverture de section circulaire est située entre ledit laser et ladite lentille à focale variable, et dans lequel ladite lentille à focale variable (14) reproduit ladite ouverture suivant une section de surface circulaire restreinte qui varie en fonction du réglage instantané de ladite commande de focale variable (15), ce par quoi le traitement chirurgical peut être correcteur de myopie.

4. Appareil selon la revendication 1 ou 2, dans lequel une ouverture allongée de largeur constante est située entre ledit laser et ladite lentille à focale variable, et dans lequel ladite lentille à focale variable (105) reproduit ladite ouverture suivant une ligne droite de largeur restreinte qui varie en fonction du réglage instantané de ladite commande de focale variable (106), ce par quoi le traitement chirurgical peut être correcteur d'astigmatisme.

5. Appareil selon la revendication 4, dans lequel ladite lentille à focale variable (105) est montée en vue de la rotation d'ensemble sélective autour de l'axe de la lentille à focale variable, ce par quoi l'orientation angulaire de ladite ligne droite peut être réglée pour concorder avec celle de la correction d'astigmatisme requise.

6. Appareil pour effectuer de la chirurgie ophtalmologique par ablation sélective de la surface antérieure de la cornée, avec pénétration dans le stroma, pour réaliser un retrait volumétrique de tissu cornéen,
   ledit appareil comprenant :
   - un moyen formant laser (13) pour produire un faisceau de sortie, de préférence dans la région de l'ultraviolet du spectre électromagnétique, et d'une intensité qui est limitée par unité de temps pour n'enlever qu'une fraction d'une profondeur d'ablation maximale prédéterminée dans le stroma ;
   - un moyen (10, 18, 20) pour diriger ledit faisceau de sortie du laser sur l'oeil qui doit être traité ; et
   - un moyen de commande (16 ; 46 ; 57 ; 89 ; 141) pour déterminer la densité de flux irradié et le temps d'exposition de l'impact du faisceau laser sur la cornée, pour effectuer ainsi une action de sculpture contrôlée sur la cornée de façon à en modifier les propriétés optiques ; et
   ledit appareil comprenant en outre :
   - un moyen de masquage à surface variable (35 ; 50 ; 62 ; 75 ; 80 ; 101, 102, 104), disposé sur la trajectoire du faisceau laser pour déterminer de façon ajustable la dimension et/ou la forme de la surface du faisceau laser au niveau de l'impact sur la cornée, et un moyen d'actionnement (40 ; 55 ; 88 ; 100) pour faire varier la surface de l'ouverture du moyen de masquage ; et

- ledit moyen de commande (16 ; 46 ; 57 ; 89 ; 141) agissant sur ledit moyen d'actionnement pour ajuster la surface du faisceau laser au niveau de l'impact sur la cornée d'une manière contrôlée au cours d'un traitement chirurgical, de telle sorte que l'action de sculpture résulte d'une variation contrôlée de la surface de la cornée qui est irradiée et d'une variation contrôlée correspondante du flux d'ablation cumulé adressé à différentes zones de la cornée.

7. Appareil selon la revendication 6, dans lequel ledit moyen de masquage comprend un diaphragme à ouverture variable.

8. Appareil selon la revendication 6 ou 7, dans lequel ledit moyen de masquage (35 ; 50) agit pour fournir une exposition de la cornée sur une zone maximale et sur une pluralité donnée de zones de forme analogue mais de surfaces inférieures, lesdites zones étant circulaires, ce par quoi le traitement chirurgical peut être correcteur de myopie.

9. Appareil selon la revendication 6 ou 7, dans lequel ledit moyen de masquage (80 ; 101, 102, 104) agit pour fournir une exposition de la cornée sur une zone maximale et sur une pluralité donnée de zones de forme analogue mais de surfaces inférieures, lesdites zones ayant la forme de rectangles allongés et étant de largeur variable, ce par quoi le traitement chirurgical peut être correcteur d'astigmatisme.

10. Appareil selon la revendication 9, dans lequel l'orientation de la direction de la longueur desdites zones est ajustable.

11. Appareil selon la revendication 6, dans lequel ledit moyen de masquage (62) agit pour fournir une exposition de la cornée sur une zone maximale et sur une pluralité donnée de zones de forme analogue mais de surfaces inférieures, lesdites zones ayant la forme d'anneaux circulaires et étant définies par un diamètre externe constant et par un diamètre interne variable, ce par quoi le traitement chirurgical peut être correcteur d'hypermétropie.

12. Appareil selon la revendication 6, dans lequel ledit moyen de masquage (75) agit pour fournir une exposition de la cornée sur une zone maximale et sur une pluralité donnée de zones de forme analogue mais de surfaces inférieures, lesdites zones ayant la forme d'anneaux circulaires et étant définies par un diamètre interne constant et par un diamètre externe variable, lequel est intermédiaire entre ledit diamètre interne et ledit diamètre externe de ladite zone maximale, ce par quoi le traitement chirurgical peut être correcteur de myopie dans un anneau de Fresnel sculpté défini par lesdits diamètres interne et externe.

13. Appareil selon la revendication 6, dans lequel ledit moyen de masquage agit pour fournir une exposition de la cornée sur une zone maximale et sur une pluralité donnée de zones de forme analogue mais de surfaces inférieures, lesdites zones ayant la forme d'anneaux circulaires, étant définies par un diamètre externe constant et par un diamètre interne qui varie jusqu'à un diamètre interne minimal fixé, ce par quoi le traitement chirurgical peut être correcteur d'hypermétropie dans un anneau de Fresnel sculpté défini par ledit diamètre externe constant et par ledit diamètre interne minimal fixé.

14. Appareil selon la revendication 6 ou selon l'une des revendications 8 à 13 en tant qu'elles sont dépendantes de la revendication 6, dans lequel ledit moyen de masquage (35 ; 50 ; 62 ; 75 ; 80) comprend une plaque opaque ayant une succession de fenêtres qui sont transparentes à la transmission à travers elles du faisceau laser et de surface changeant progressivement, et ledit moyen d'actionnement (40 ; 55 ; 88) est commandé par microprocesseur pour amener lesdites fenêtres en alignement successif avec l'axe du faisceau laser.

15. Appareil pour effectuer de la chirurgie ophtalmologique par ablation sélective de la surface antérieure de la cornée, avec pénétration dans le stroma, pour réaliser un retrait volumétrique de tissu cornéen,
    ledit appareil comprenant :
    - un moyen formant laser (13) pour produire un faisceau de sortie, de préférence dans la région de l'ultraviolet du spectre électromagnétique, et d'une intensité qui est limitée par unité de temps pour n'enlever qu'une fraction d'une profondeur d'ablation maximale prédéterminée dans le stroma ;
    - un moyen (10, 18, 20) pour diriger ledit faisceau de sortie du laser sur l'oeil qui doit être traité ; et
    - un moyen de commande (16 ; 46 ; 57 ; 89 ; 141) pour déterminer la densité de flux irradié et le temps d'exposition de l'impact du faisceau laser sur la cornée, pour effectuer ainsi une action de sculpture contrôlée sur la cornée de façon à en modifier les propriétés optiques ; et

ledit appareil comprenant en outre :
- un moyen réflecteur (135 ; 150 ; 162 ; 175), disposé sur la trajectoire du faisceau laser pour limiter de façon variable la surface du faisceau laser au niveau de l'impact sur la cornée et un moyen d'actionnement (140 ; 155) pour faire varier la surface réflectrice dudit moyen réflecteur ; et
- ledit moyen de commande (16 ; 46 ; 57 ; 89 ; 141) agissant sur ledit moyen d'actionnement pour ajuster la surface du faisceau laser au niveau de l'impact sur la cornée d'une manière contrôlée au cours d'un traitement chirurgical, de telle sorte que l'action de sculpture résulte d'une variation contrôlée de la surface de la cornée qui est irradiée et d'une variation contrôlée correspondante du flux d'ablation cumulé adressé à différentes zones de la cornée.

16. Appareil selon la revendication 15, dans lequel ledit moyen réflecteur (135 ; 150) agit pour fournir une exposition de la cornée sur une zone circulaire centrale et sur une pluralité donnée de zones de forme analogue mais de surfaces supérieures, lesdites zones étant concentriques, ce par quoi le traitement chirurgical peut être correcteur de myopie.

17. Appareil selon la revendication 15, dans lequel ledit moyen réflecteur (162) agit pour fournir une exposition de la cornée sur une zone maximale et sur une pluralité donnée de zones de forme analogue mais de surfaces inférieures, lesdites zones ayant la forme d'anneaux circulaires et étant définies par un diamètre externe constant et par un diamètre interne variable, ce par quoi le traitement chirurgical peut être correcteur d'hypermétropie.

18. Appareil selon la revendication 15, dans lequel ledit moyen réflecteur agit pour fournir une exposition de la cornée sur une zone ayant la forme d'un rectangle allongé étroit, centrée sur l'axe optique de l'oeil et couvrant une zone maximale, plusieurs tels moyens réflecteurs agissant en outre pour fournir une exposition de la cornée sur des zones de forme analogue mais de surfaces supérieures, lesdites zones ayant la forme d'un rectangle allongé et de largeur variable, qui est symétrique par rapport à la direction de la longueur de ladite zone étroite, ce par quoi le traitement chirurgical peut être correcteur d'astigmatisme.

19. Appareil selon la revendication 18, dans lequel l'orientation de la direction de la longueur desdites zones est ajustable.

20. Appareil selon la revendication 15, dans lequel ledit moyen réflecteur (175) agit pour fournir une exposition de la cornée sur une zone maximale et sur une pluralité donnée de zones de forme analogue mais de surfaces inférieures, lesdites zones ayant la forme d'anneaux circulaires et étant définies par un diamètre interne constant et par un diamètre externe variable, lequel est intermédiaire entre ledit diamètre interne et ledit diamètre externe de ladite zone maximale, ce par quoi le traitement chirurgical peut être correcteur de myopie dans un anneau de Fresnel sculpté défini par lesdits diamètres interne et externe.

21. Appareil selon la revendication 15, dans lequel ledit moyen réflecteur agit pour fournir une exposition de la cornée sur une zone maximale et sur une pluralité données de zones de forme analogue mais de surfaces inférieures, lesdites zones ayant la forme d'anneaux circulaires, étant définies par un diamètre externe constant et par un diamètre interne qui varie jusqu'à un diamètre interne minimal fixé, ce par quoi le traitement chirurgical peut être correcteur d'hypermétropie dans un anneau de Fresnel sculpté défini par ledit diamètre externe constant et par ledit diamètre interne minimal fixé.

22. Appareil selon l'une des revendications 15 à 21, dans lequel ledit moyen réflecteur (135 ; 150 ; 162 ; 175) comprend une plaque transparente ayant une succession d'éléments réflecteurs sur l'une de ses surfaces, lesdits éléments réflecteurs ayant une surface changeant progressivement, et ledit moyen d'actionnement (140 ; 155) est commandé par microprocesseur pour amener lesdits éléments réflecteurs en alignement successif avec l'axe du faisceau laser.

23. Appareil selon la revendication 15, dans lequel ledit moyen réflecteur est un diaphragme à ouverture variable caractérisé par un côté réflecteur orienté pour réfléchir le faisceau laser dans une zone annulaire continue à la périphérie, entourant l'ouverture du diaphragme.

24. Appareil selon l'une des revendications 15 à 22, dans lequel l'incidence du faisceau laser sur ledit moyen réflecteur est à 45 degrés, et dans lequel la zone réflectrice est elliptique avec un rapport grand axe à petit axe de $\sqrt{2}:1$, l'incidence du faisceau laser étant centrée sur l'ellipse et à 45 degrés par rapport à son grand axe.

**25.** Procédé, à l'exclusion de l'application de ce procédé sur le corps humain et/ou animal vivant, de sculpture de la cornée par ablation sélective de la surface cornéenne avec pénétration dans le stroma, de façon à réaliser un retrait volumétrique de tissu cornéen, ledit procédé comprenant les opérations consistant à :

- utiliser un moyen formant laser (13) pour produire un faisceau de sortie, de préférence, dans la région de l'ultraviolet du spectre électromagnétique et d'une intensité qui est limitée par unité de temps, pour n'enlever qu'une fraction d'une profondeur d'ablation maximale prédéterminée dans le stroma ;
- diriger ledit faisceau de sortie du laser à travers une lentille à focale variable (14 ; 105) disposée sur la trajectoire du faisceau laser, sur la cornée qui doit être sculptée ; et
- commander la densité de flux irradié et le temps d'exposition de l'impact du faisceau laser sur la cornée, pour effectuer ainsi une action de sculpture contrôlée sur la cornée, de façon à en modifier les propriétés optiques, une telle commande étant effectuée par ajustement de la lentille à focale variable (14 ; 105) de façon à ajuster la dimension de la surface du faisceau laser au niveau de l'impact sur la cornée, de telle sorte que l'action de sculpture résulte d'une variation contrôlée de la surface de la cornée qui est irradiée et d'une variation contrôlée correspondante du flux d'ablation cumulé adressé aux différentes zones de la cornée.

**26.** Procédé, à l'exclusion de l'application de ce procédé sur le corps humain et/ou animal vivant, de sculpture de la cornée par ablation sélective de la surface cornéenne avec pénétration dans le stroma, de façon à réaliser un retrait volumétrique de tissu cornéen, ledit procédé comprenant les opérations consistant à :

- utiliser un moyen formant laser (13) pour produire un faisceau de sortie, de préférence, dans la région de l'ultraviolet du spectre électromagnétique et d'une intensité qui est limitée par unité de temps, pour n'enlever qu'une fraction d'une profondeur d'ablation maximale prédéterminée dans le stroma ;
- diriger ledit faisceau de sortie du laser sur la cornée qui doit être sculptée ; et
- commander la densité de flux irradié et le temps d'exposition de l'impact du faisceau laser sur la cornée, pour effectuer ainsi une action de sculpture contrôlée sur la cornée, de façon à en modifier les propriétés optiques, une telle commande étant effectuée par ajustement de la dimension et/ou de la forme de la surface du faisceau laser au niveau de l'impact sur la cornée, de telle sorte que l'action de sculpture résulte d'une variation contrôlée de la surface de la cornée qui est irradiée et d'une variation contrôlée correspondante du flux d'ablation cumulé adressé aux différentes zones de la cornée ;
- l'ajustement de la surface du faisceau laser au niveau de l'impact sur la cornée étant effectué par utilisation sélective d'un moyen de masquage du faisceau à surface variable (35 ; 50 ; 62 ; 75 ; 80 ; 101, 102, 104) et en faisant varier la surface de l'ouverture du moyen de masquage afin de définir une pluralité de différentes dimensions et/ou formes données de surface de faisceau laser au niveau de l'impact sur la cornée.

**27.** Procédé, à l'exclusion de l'application de ce procédé sur le corps humain et/ou animal vivant, de sculpture de la cornée par ablation sélective de la surface cornéenne avec pénétration dans le stroma, de façon à réaliser un retrait volumétrique de tissu cornéen, ledit procédé comprenant les opérations consistant à :

- utiliser un moyen formant laser (13) pour produire un faisceau de sortie, de préférence, dans la région de l'ultraviolet du spectre électromagnétique et d'une intensité qui est limitée par unité de temps, pour n'enlever qu'une fraction d'une profondeur d'ablation maximale prédéterminée dans le stroma ;
- diriger ledit faisceau de sortie du laser sur la cornée qui doit être sculptée ; et
- commander la densité de flux irradié et le temps d'exposition de l'impact du faisceau laser sur la cornée, pour effectuer ainsi une action de sculpture contrôlée sur la cornée, de façon à en modifier les propriétés optiques, une telle commande étant effectuée par ajustement de la dimension et/ou de la forme de la surface du faisceau laser au niveau de l'impact sur la cornée, de telle sorte que l'action de sculpture résulte d'une variation contrôlée de la surface de la cornée qui est irradiée et d'une variation contrôlée correspondante du flux d'ablation cumulé adressé aux différentes zones de la cornée ;
- l'ajustement de la surface du faisceau laser au niveau de l'impact sur la cornée étant effectué par utilisation sélective d'un moyen de réflexion du faisceau (135 ; 150 ; 162 ; 175) définissant une pluralité de différentes dimensions et/ou formes données de surface de faisceau laser au niveau de l'impact sur la cornée.

28. Procédé selon l'une quelconque des revendications 25 à 27, dans lequel l'action de sculpture est effectuée à l'intérieur d'une zone centrale prédéterminée de la cornée et avec la cornée soumise à la plus grande densité d'exposition du faisceau laser au centre de ladite zone centrale et à une densité d'exposition qui diminue avec l'augmentation du rayon jusqu'aux régions externes de ladite zone centrale.

29. Procédé selon la revendication 26 ou 27, dans lequel l'action de sculpture est effectuée à l'intérieur d'une zone centrale prédéterminée de la cornée et avec la cornée soumise à la plus faible densité d'exposition du faisceau laser au centre de ladite zone centrale et à une densité d'exposition qui augmente avec l'augmentation du rayon jusqu'aux régions externes de ladite zone centrale.

30. Procédé selon l'une quelconque des revendications 25 à 27, dans lequel le faisceau laser a la forme d'un rectangle allongé au niveau de l'impact sur la cornée, et l'action de sculpture est effectuée avec l'axe d'allongement du faisceau fixé dans une orientation prédéterminée et en faisant varier la largeur du faisceau rectangulaire transversalement à son axe précité, de telle sorte qu'une région centrale allongée de la cornée est soumise à la plus grande densité d'exposition du faisceau laser et à une densité d'exposition qui diminue avec une augmentation du décalage latéral sur l'un ou l'autre des côtés de ladite région centrale allongée.

31. Procédé selon la revendication 30, dans lequel la fixation de l'axe d'allongement du faisceau laser est effectuée automatiquement en fonction du diagnostic des caractéristiques optiques de la cornée qui doit être sculptée.

32. Procédé selon l'une quelconque des revendications 25 à 31, dans lequel l'action de sculpture est effectuée en une série d'opérations de façon à réaliser une recaractérisation de Fresnel de la surface cornéenne.

EP 0 207 648 B2

FIG. 1.

LASER
FIXATION
POWER SUPPLY
PROGRAMMABLE EXPOSURE & ZOOM DRIVE
ZOOM LENS
CLAMP MEANS

FIG. 2.

VACUUM
RIGID CONNECTION

FIG. 3.

FIG. 4.

FIG. 5.

## FIG. 6.

LASER PULSE SYNCH

45

40

X-Y COORDINATE INDEX DRIVE

46

MICROPROCESSOR (PROGRAMMABLE SHOT ALLOCATION)

11

## FIG. 7.

## FIG. 8.

50

55

LASER-PULSE SYNCH

56

ROTARY DRIVE SIGNAL GEN.

58

57

MICROPROCESSOR (PROGRAMMABLE SHOT ALLOCATION)

11

## FIG. 9.

50

## FIG. 10.

## FIG. II.

FIG. 14.

FIG. 12.

FIG. 15.

FIG. 13.

POSTERIOR
CORNEAL
SURFACE

AXIS    1    2    3
mm RADIUS

SLIDE DRIVE
SIGNAL
GEN.

PULSE
SYNCH

MICROPROCESSOR
(PROGRAMMABLE
SHOT ALLOCATION)

FIG. 17.

FIG. 18.

FIG. 16.

# FIG. 19.

SLOT-WIDTH
DRIVE
MEANS

FROM
MICROPROCESSOR

# FIG. 20.

FIG. 21.

FIG. 22.

FIG. 24.

FIG. 23.

FIG. 25.

# FIG. 26.

# FIG. 27.

# FIG. 28.